# EUROPEAN PATENT SPECIFICATION

(11) **EP 2 721 413 B1**
(45) Date of publication and mention of the grant of the patent: **03.02.2016**
(21) Application number: 12733615.4
(22) Date of filing: 14.06.2012
(51) Int. Cl.: G01N 33/564, G01N 33/576

(54) **BIOMARKERS FOR AUTOIMMUNE LIVER DISEASES AND USES THEREOF**
BIOMARKER FÜR AUTOIMMUNKRANKHEITEN DES LEBERS UND DEREN VERWENDUNG
BIOMARQUEURS DES MALADIES AUTOIMMUNES DE FOIE ET LEUR UTILISATION

(30) Priority: 16.06.2011 EP 11170252
(43) Date of publication of application: 23.04.2014
(73) Proprietor: Istituto Nazionale Di Genetica Molecolare-INGM, 20122 Milano (IT)
(72) Inventor: BOMBACI, Mauro, I-20147 Milano (MI) (IT); ABRIGNANI, Sergio, I-53040 Rapolano Terme (SI) (IT); ZINGARETTI, Chiara, I-48121 Ravenna (RA) (IT)
(74) Representative: Capasso, Olga
(86) International application number: PCT/EP2012/061313
(87) International publication number: WO 2012/172004

(56) References cited:
- WO-A2-2009/081201
- G DALEKOS: "Autoantibodies and defined target autoantigens in autoimmune hepatitis: an overview", EUROPEAN JOURNAL OF INTERNAL MEDICINE, vol. 13, no. 5, 1 August 2002 (2002-08-01) , pages 293-303, XP055006755, ISSN: 0953-6205, DOI: 10.1016/S0953-6205(02)00089-4
- D E J JONES: "Autoantigens in primary biliary cirrhosis", JOURNAL OF CLINICAL PATHOLOGY, vol. 53, no. 11, 1 November 2000 (2000-11-01), pages 813-821, XP055006756, ISSN: 0021-9746, DOI: 10.1136/jcp.53.11.813
- ZHANG J-L ET AL: "The high-affinity interaction of human IL-4 and the receptor alpha chain is constituted by two independent binding clusters", JOURNAL OF MOLECULAR BIOLOGY, ACADEMIC PRESS, UNITED KINGDOM, vol. 315, no. 3, 18 January 2002 (2002-01-18), pages 399-407, XP004469315, ISSN: 0022-2836, DOI: 10.1006/JMBI.2001.5243

### SEQUENCE LISTING

<110> Istituto Nazionale di Genetica Molecolare - INGM
<120> BIOMARKERS FOR AUTOIMMUNE LIVER DISEASES AND USES THEREOF
<130> PCT117422
<160> 25
<170> PatentIn version 3.5
<210> 1
   <211> 97
   <212> PRT
   <213> Homo sapiens
<400> 1
   Pro Arg Ala Pro Gly Asn Leu Thr Val His Thr Asn Val Ser Asp Thr
   1               5                   10                  15
   Leu Leu Leu Thr Trp Ser Asn Pro Tyr Pro Pro Asp Asn Tyr Leu Tyr
               20                  25                  30
   Asn His Leu Thr Tyr Ala Val Asn Ile Trp Ser Glu Asn Asp Pro Ala
           35                  40                  45
   Asp Phe Arg Ile Tyr Asn Val Thr Tyr Leu Glu Pro Ser Leu Arg Ile
       50                  55                  60
   Ala Ala Ser Thr Leu Lys Ser Gly Ile Ser Tyr Arg Ala Arg Val Arg
   65                  70                  75                  80
   Ala Trp Ala Gln Cys Tyr Asn Thr Thr Trp Ser Glu Trp Ser Pro Ser
                   85                  90                  95
   Thr
<210> 2
   <211> 175
   <212> PRT
   <213> Homo sapiens
<400> 2
   Ser Asn Trp Gly Cys Tyr Gly Asn Ile Gln Ser Leu Asp Thr Pro Gly
   1               5                   10                  15
   Ala Ser Cys Gly Ile Gly Arg Arg His Gly Leu Asn Tyr Cys Gly Val
               20                  25                  30
   Arg Ala Ser Glu Arg Leu Ala Glu Ile Asp Met Pro Tyr Leu Leu Lys
           35                  40                  45
   Tyr Gln Pro Met Met Gln Thr Ile Gly Gln Lys Tyr Cys Met Asp Pro
       50                  55                  60
   Ala Val Ile Ala Gly Val Leu Ser Arg Lys Ser Pro Gly Asp Lys Ile
   65                  70                  75                  80
   Leu Val Asn Met Gly Asp Arg Thr Ser Met Val Gln Asp Pro Gly Ser
                   85                  90                  95
   Gln Ala Pro Thr Ser Trp Ile Ser Glu Ser Gln Val Ser Gln Thr Thr
               100                 105                 110
   Glu Val Leu Thr Thr Arg Ile Lys Glu Ile Gln Arg Arg Phe Pro Thr
           115                 120                 125
   Trp Thr Pro Asp Gln Tyr Leu Arg Gly Gly Leu Cys Ala Tyr Ser Gly
       130                 135                 140
   Gly Ala Gly Tyr Val Arg Ser Ser Gln Asp Leu Ser Cys Asp Phe Cys
   145                 150                 155                 160
   Asn Asp Val Leu Ala Arg Ala Lys Tyr Leu Lys Arg His Gly Phe
                   165                 170                 175
<210> 3
   <211> 237
   <212> PRT
   <213> Homo sapiens
<400> 3
   Met Phe Val Asp Asn Arg Ile Gln Lys Ser Met Leu Leu Asp Leu Asn
   1 5 10 15
   Lys Glu Ile Met Asn Glu Leu Gly Val Thr Val Val Gly Asp Ile Ile
   20 25 30
   Ala Ile Leu Lys His Ala Lys Val Val His Arg Gln Asp Met Cys Lys
   35 40 45
   Ala Ala Thr Glu Ser Val Pro Cys Ser Pro Ser Pro Leu Ala Gly Glu
   50 55 60
   Ile Arg Arg Gly Thr Ser Ala Ala Ser Arg Met Ile Thr Asn Ser Leu
   65 70 75 80
   Asn His Asp Ser Pro Pro Ser Thr Pro Pro Arg Arg Pro Asp Thr Ser
   85 90 95
   Thr Ser Lys Ile Ser Val Thr Val Ser Asn Lys Met Ala Ala Lys Ser
   100 105 110
   Ala Lys Ala Thr Ala Ala Leu Ala Arg Arg Glu Glu Glu Ser Leu Ala
   115 120 125
   Val Pro Ala Lys Arg Arg Arg Val Thr Ala Glu Met Glu Gly Lys Tyr
   130 135 140
   Val Ile Asn Met Pro Lys Gly Thr Thr Pro Arg Thr Arg Lys Ile Leu
   145 150 155 160
   Glu Gln Gln Gln Ala Ala Lys Gly Leu His Arg Thr Ser Val Phe Asp
   165 170 175
   Arg Leu Gly Ala Glu Thr Lys Ala Asp Thr Thr Thr Gly Ser Lys Pro
   180 185 190
   Thr Gly Val Phe Ser Arg Leu Gly Ala Thr Pro Glu Thr Asp Glu Asp
   195 200 205
   Leu Ala Trp Asp Ser Asp Asn Asp Ser Ser Ser Ser Val Leu Gln Tyr
   210 215 220
   Ala Gly Val Leu Lys Lys Leu Gly Arg Gly Pro Ala Lys
   225 230 235
<210> 4
   <211> 79
   <212> PRT
   <213> Homo sapiens
<400> 4
   Ser Gln Gly Val Cys Ser Lys Gln Thr Leu Val Val Pro Leu His Tyr
   1               5                   10                  15
   Asn Glu Ser Tyr Ser Gln Pro Val Tyr Lys Pro Tyr Leu Thr Leu Cys
               20                  25                  30
   Ala Gly Arg Arg Ile Cys Ser Thr Tyr Arg Thr Met Tyr Arg Val Met
           35                  40                  45
   Trp Arg Glu Val Arg Arg Glu Val Gln Gln Thr His Ala Val Cys Cys
       50                  55                  60
   Gln Gly Trp Lys Lys Arg His Pro Gly Ala Leu Thr Cys Glu Ala
   65                  70                  75
<210> 5
   <211> 76
   <212> PRT
   <213> Homo sapiens
<400> 5
   His Glu Ala His Lys Thr Ser Leu Ser Ser Trp Lys His Asp Gln Asp
   1               5                   10                  15
   Trp Ala Asn Val Ser Asn Met Thr Phe Ser Asn Gly Lys Leu Arg Val
               20                  25                  30
   Lys Gly Ile Tyr Tyr Arg Asn Ala Asp Ile Cys Ser Arg His Arg Val
           35                  40                  45
   Thr Ser Ala Gly Leu Thr Leu Gln Asp Leu Gln Leu Trp Cys Asn Leu
       50                  55                  60
   Arg Ser Val Ala Arg Gly Gln Ile Pro Ser Thr Leu
   65                  70                  75
<210> 6
   <211> 101
   <212> PRT
   <213> Homo sapiens
<400> 6
   Gly Val Ala Glu Phe His Met Ser Leu Thr Val Ser Cys Pro Asp Pro
   1               5                   10                  15
   Thr Pro Ser Thr Asp Pro Gln Gly Arg His Asn Arg Glu Pro Ile Leu
               20                  25                  30
   Gly Arg Asp Asp Asp Phe Met Cys Lys Gln Val Lys Phe Arg Met Cys
           35                  40                  45
   Val Val Gly Arg Asp Gly Asn Ala Gln Ser Ser Val Arg Tyr Thr Gly
       50                  55                  60
   Pro Leu Tyr Arg Arg Lys Ile Arg Thr Glu Phe Leu Phe Val Val Phe
   65                  70                  75                  80
   Leu Leu Glu Thr Arg Glu Leu Lys Pro Gln Val Asn Lys Asn Val Gln
                   85                  90                  95
   Gly Thr Arg Pro Ser
               100
<210> 7
   <211> 92
   <212> PRT
   <213> Homo sapiens
<400> 7
<210> 8
   <211> 92
   <212> PRT
   <213> Homo sapiens
<400> 8
   Ala Thr Gly Ile Leu Ile Cys Met Thr Lys Asn Leu Glu Ser Val His
   1               5                   10                  15
   Ser Ile Val Leu Ala His Ser Cys Tyr His His Glu Asn Lys Pro Arg
               20                  25                  30
   Pro Asp Cys Cys Phe Gln Gln Lys Ile Arg Asp Thr Lys Ser Lys Val
           35                  40                  45
   Glu Leu Pro Arg His Ala His Ala Arg Leu Thr Asn Pro Gln Leu Thr
       50                  55                  60
   His Arg Ser Met Lys Ile Asn Asp Cys Cys Ile Lys Pro Leu Arg Phe
   65                  70                  75                  80
   Gly Val Thr Cys Tyr Ala Ala Phe Cys Asp Asn Asn
                   85                  90
<210> 9
   <211> 237
   <212> PRT
   <213> Homo sapiens
<400> 9
   Ala Arg Glu Glu Glu Ile Thr Pro Val Val Ser Ile Ala Tyr Lys Val
   1               5                   10                  15
   Leu Glu Val Phe Pro Lys Gly Arg Trp Val Leu Ile Thr Cys Cys Ala
               20                  25                  30
   Pro Gln Pro Pro Pro Pro Ile Thr Tyr Ser Leu Cys Gly Thr Lys Asn
           35                  40                  45
   Ile Lys Val Ala Lys Lys Val Val Lys Thr His Glu Pro Ala Ser Phe
       50                  55                  60
   Asn Leu Asn Val Thr Leu Lys Ser Ser Pro Asp Leu Leu Thr Tyr Phe
   65                  70                  75                  80
   Cys Trp Ala Ser Ser Thr Ser Gly Ala His Val Asp Ser Ala Arg Leu
                   85                  90                  95
   Gln Met His Trp Glu Leu Trp Ser Lys Pro Val Ser Glu Leu Arg Ala
               100                 105                 110
   Asn Phe Thr Leu Gln Asp Arg Gly Ala Gly Pro Arg Val Glu Met Ile
           115                 120                 125
   Cys Gln Ala Ser Ser Gly Ser Pro Pro Ile Thr Asn Ser Leu Ile Gly
       130                 135                 140
   Lys Asp Gly Gln Val His Leu Gln Gln Arg Pro Cys His Arg Gln Pro
   145                 150                 155                 160
   Ala Asn Phe Ser Phe Leu Pro Ser Gln Thr Ser Asp Trp Phe Trp Cys
                   165                 170                 175
   Gln Ala Ala Asn Asn Ala Asn Val Gln His Ser Ala Leu Thr Val Val
               180                 185                 190
   Pro Pro Gly Gly Asp Gln Lys Met Glu Asp Trp Gln Gly Pro Leu Glu
           195                 200                 205
   Ser Pro Ile Leu Ala Leu Pro Leu Tyr Arg Ser Thr Arg Arg Leu Ser
       210                 215                 220
   Glu Glu Glu Phe Gly Gly Phe Arg Ile Gly Asn Gly Glu
   225                 230                 235
<210> 10
   <211> 124
   <212> PRT
   <213> Homo sapiens
<400> 10
   Val Ala Lys Lys Val Val Lys Thr His Glu Pro Ala Ser Phe Asn Leu
   1               5                   10                  15
   Asn Val Thr Leu Lys Ser Ser Pro Asp Leu Leu Thr Tyr Phe Cys Trp
               20                  25                  30
   Ala Ser Ser Thr Ser Gly Ala His Val Asp Ser Ala Arg Leu Gln Met
           35                  40                  45
   His Trp Glu Leu Trp Ser Lys Pro Val Ser Glu Leu Arg Ala Asn Phe
       50                  55                  60
   Thr Leu Gln Asp Arg Gly Ala Gly Pro Arg Val Glu Met Ile Cys Gln
   65                  70                  75                  80
   Ala Ser Ser Gly Ser Pro Pro Ile Thr Asn Ser Leu Ile Gly Lys Asp
                   85                  90                  95
   Gly Gln Val His Leu Gln Gln Arg Pro Cys His Arg Gln Pro Ala Asn
               100                 105                 110
   Phe Ser Phe Leu Pro Ser Gln Thr Ser Asp Trp Phe
           115                 120
<210> 11
   <211> 237
   <212> PRT
   <213> Homo sapiens
<400> 11
   Ala Gln Tyr Ser Ser Asp Arg Cys Ser Trp Lys Gly Ser Gly Leu Thr
   1               5                   10                  15
   His Glu Ala His Arg Lys Glu Val Glu Gln Val Tyr Leu Arg Cys Ala
               20                  25                  30
   Ala Gly Ala Val Glu Trp Met Tyr Pro Thr Gly Ala Leu Ile Val Asn
           35                  40                  45
   Leu Arg Pro Asn Thr Phe Ser Pro Ala Arg His Leu Thr Val Cys Ile
   50 55 60
   Arg Ser Phe Thr Asp Ser Ser Gly Ala Asn Ile Tyr Leu Glu Lys Thr
   65 70 75 80
   Gly Glu Leu Arg Leu Leu Val Pro Asp Gly Asp Gly Arg Pro Gly Arg
   85 90 95
   Val Gln Cys Phe Gly Leu Glu Gln Gly Gly Leu Phe Val Glu Ala Thr
   100 105 110
   Pro Gln Gln Asp Ile Gly Arg Arg Thr Thr Gly Phe Gln Tyr Glu Leu
   115 120 125
   Val Arg Arg His Arg Ala Ser Asp Leu His Glu Leu Ser Ala Pro Cys
   130 135 140
   Arg Pro Cys Ser Asp Thr Glu Val Leu Leu Ala Val Cys Thr Ser Asp
   145 150 155 160
   Phe Ala Val Arg Gly Ser Ile Gln Gln Val Thr His Glu Pro Glu Arg
   165 170 175
   Gln Asp Ser Ala Ile His Leu Arg Val Ser Arg Leu Tyr Arg Gln Lys
   180 185 190
   Ser Arg Val Phe Glu Pro Val Pro Glu Gly Asp Gly His Trp Gln Gly
   195 200 205
   Arg Val Arg Thr Leu Leu Glu Cys Gly Val Arg Pro Gly His Gly Asp
   210 215 220
   Phe Leu Phe Thr Gly His Met His Phe Gly Glu Ala Arg
   225 230 235
<210> 12
   <211> 95
   <212> PRT
   <213> Homo sapiens
<400> 12
   Met Phe Gly Val Leu Glu Gly Ala Gln Ala Asn Ser Glu Asn Trp Ile
   1 5 10 15
   Ala Pro Ser Gly Pro Trp Ala Leu Gly Leu Trp Ser Ser Leu Tyr Phe
   20 25 30
   Leu Leu Phe Ser Thr Leu Glu Gly Arg Gly Gly Arg Val Leu Ser Gln
           35                  40                  45
   Ser Cys Ser Met Ala Val Ala Ala Ala Ser Trp Ile Ser Arg Glu Asn
       50                  55                  60
   Ala Arg Ser Val Lys Arg Ser Tyr Met Gln Ser Ser Pro Gln Arg Pro
   65                  70                  75                  80
   Lys Glu Pro Arg Asn Gln Arg Thr Ser His Thr Thr Pro Val Cys
                   85                  90                  95
<210> 13
   <211> 205
   <212> PRT
   <213> Homo sapiens
<400> 13
   Ser Gly Phe Thr Ala Leu His Trp Ala Ala Lys Ser Gly Asp Gly Glu
   1               5                   10                  15
   Met Ala Leu Gln Leu Val Glu Val Ala Arg Arg Ser Gly Ala Pro Val
               20                  25                  30
   Asp Val Asn Ala Arg Ser His Gly Gly Tyr Thr Pro Leu His Leu Ala
           35                  40                  45
   Ala Leu His Gly His Glu Asp Ala Ala Val Leu Leu Val Val Arg Leu
       50                  55                  60
   Gly Ala Gln Val His Val Arg Asp His Ser Gly Arg Arg Ala Tyr Gln
   65                  70                  75                  80
   Tyr Leu Arg Pro Gly Ser Ser Tyr Ala Leu Arg Arg Leu Leu Gly Asp
                   85                  90                  95
   Pro Gly Leu Arg Gly Thr Thr Glu Pro Asp Ala Thr Gly Gly Gly Ser
               100                 105                 110
   Gly Ser Leu Ala Ala Arg Arg Pro Val Gln Val Ala Ala Thr Ile Leu
           115                 120                 125
   Ser Ser Thr Thr Ser Ala Phe Leu Gly Val Leu Ala Asp Asp Leu Met
       130                 135                 140
   Leu Gln Asp Leu Ala Arg Gly Leu Lys Lys Ser Ser Ser Phe Ser Lys
   145                 150                 155                 160
   Phe Leu Ser Ala Ser Pro Met Ala Pro Arg Lys Lys Thr Lys Ile Arg
                   165                 170                 175
   Gly Gly Leu Pro Ala Phe Ser Glu Ile Ser Arg Arg Pro Thr Pro Gly
               180                 185                 190
   Pro Leu Ala Gly Leu Val Pro Ser Phe Pro Pro Thr Thr
           195                 200                 205
<210> 14
   <211> 125
   <212> PRT
   <213> Homo sapiens
<400> 14
   Ser Leu Ile Val Phe Met Glu Gln Val His Arg Gly Ile Lys Gly Leu
   1               5                   10                  15
   Val Arg Asp Ser His Gly Lys Gly Ile Pro Asn Ala Ile Ile Ser Val
               20                  25                  30
   Glu Gly Ile Asn His Asp Ile Arg Thr Ala Asn Asp Gly Asp Tyr Trp
           35                  40                  45
   Arg Leu Leu Asn Pro Gly Glu Tyr Val Val Thr Ala Lys Ala Glu Gly
       50                  55                  60
   Phe Thr Ala Ser Thr Lys Asn Cys Met Val Gly Tyr Asp Met Gly Ala
   65                  70                  75                  80
   Thr Arg Cys Asp Phe Thr Leu Ser Lys Thr Asn Met Ala Arg Ile Arg
                   85                  90                  95
   Glu Ile Met Glu Lys Phe Gly Lys Gln Pro Val Ser Leu Pro Ala Arg
               100                 105                 110
   Arg Leu Lys Leu Arg Gly Gln Lys Arg Arg Gln Arg Gly
           115                 120                 125
<210> 15
   <211> 99
   <212> PRT
   <213> Homo sapiens
<400> 15
   Gly Ser Leu Ser Pro Thr Lys Tyr Asn Leu Leu Glu Leu Lys Glu Ser
   1               5                   10                  15
   Cys Ile Arg Asn Gln Asp Cys Glu Thr Gly Cys Cys Gln Arg Ala Pro
               20                  25                  30
   Asp Asn Cys Glu Ser His Cys Ala Glu Lys Gly Ser Glu Gly Ser Leu
           35                  40                  45
   Cys Gln Thr Gln Val Phe Phe Gly Gln Tyr Arg Ala Cys Pro Cys Leu
       50                  55                  60
   Arg Asn Leu Thr Cys Ile Tyr Ser Lys Asn Glu Lys Trp Leu Ser Ile
   65                  70                  75                  80
   Ala Tyr Gly Arg Cys Gln Lys Ile Gly Arg Gln Lys Leu Ala Lys Lys
                   85                  90                  95
   Met Phe Phe
<210> 16
   <211> 98
   <212> PRT
   <213> Homo sapiens
<400> 16
   Ser Gln Leu Glu Leu Ile Asp Leu Ser Ser Asn Pro Phe His Cys Asp
   1               5                   10                  15
   Cys Gln Leu Leu Pro Leu His Arg Trp Leu Thr Gly Leu Asn Leu Arg
               20                  25                  30
   Val Gly Ala Thr Cys Ala Thr Pro Pro Asn Ala Arg Gly Gln Arg Val
           35                  40                  45
   Lys Ala Ala Ala Ala Val Phe Glu Asp Cys Pro Gly Trp Ala Ala Arg
       50                  55                  60
   Lys Ala Lys Arg Thr Pro Ala Ser Arg Pro Ser Ala Arg Arg Thr Pro
   65                  70                  75                  80
   Ile Lys Gly Arg Gln Cys Gly Ala Asp Lys Val Gly His Gly Ala Gly
                   85                  90                  95
   Gly Val
<210> 17
   <211> 112
   <212> PRT
   <213> Homo sapiens
<210> 18
   <211> 64
   <212> PRT
   <213> Homo sapiens
<400> 18
   Ala Thr Lys Leu Val Thr Cys Pro Ala Pro Arg Gln Phe Ala Val Gly
   1               5                   10                  15
   Ala Phe Thr Ala Ala Gly Arg Ala Trp Leu Phe Ala Pro Ser Leu Gly
               20                  25                  30
   Ala Ser Phe Ser Lys Leu Arg Ser Gln Gln Arg Ser Arg Asp Phe Arg
           35                  40                  45
   Gly Arg Leu Phe Leu Arg Ala Glu Arg Arg Ala Gly Gly Phe Thr Ser
       50                  55                  60
<210> 19
   <211> 237
   <212> PRT
   <213> Homo sapiens
<400> 19
   Ser Ser Asn Leu Glu Gly Arg Thr Lys Ser Val Ile Arg Gln Thr Gly
   1               5                   10                  15
   Ser Ser Ala Glu Ile Thr Cys Asp Leu Ala Glu Gly Ser Thr Gly Tyr
               20                  25                  30
   Ile His Trp Tyr Leu His Gln Glu Gly Lys Ala Pro Gln Arg Leu Leu
           35                  40                  45
   Tyr Tyr Asp Ser Tyr Thr Ser Ser Val Val Leu Glu Ser Gly Ile Ser
       50                  55                  60
   Pro Gly Lys Tyr Asp Thr Tyr Gly Ser Thr Arg Lys Asn Leu Arg Met
   65                  70                  75                  80
   Ile Leu Arg Asn Leu Ile Glu Asn Asp Ser Gly Val Tyr Tyr Cys Ala
                   85                  90                  95
   Thr Trp Asp Gly His Ser Asp Ser Asp Pro Pro Tyr Thr Thr Leu Lys
               100                 105                 110
   Thr Cys Leu Val Ala Ala Ser Pro Arg Glu Glu Gly Met Arg Trp Ala
           115                 120                 125
   Leu Leu Val Leu Leu Ala Phe Leu Ser Pro Ile Pro Ala Pro Pro Thr
       130                 135                 140
   Val Phe Cys Ala Arg Asp His Phe Leu Val Glu Trp Asp Leu Ser Phe
   145                 150                 155                 160
   Glu Arg Ile Phe Tyr Ser Phe Ser Ser Gly Pro Phe Pro Ser Lys Ala
                   165                 170                 175
   Pro Glu Arg Lys Ala Cys Ser Gln Lys Ser Ser Asn Leu Glu Gly Arg
               180                 185                 190
   Met Lys Ser Val Thr Arg Pro Thr Gly Ser Ser Ala Glu Ile Thr Cys
           195                 200                 205
   Asp Leu Thr Val Ile Asn Ala Val Tyr Ile His Trp Tyr Leu Gln Gln
       210                 215                 220
   Glu Gly Lys Thr Pro Gln His Leu Leu His Tyr Asp Val
   225                 230                 235
<210> 20
   <211> 145
   <212> PRT
   <213> Homo sapiens
<400> 20
   Ala Gly Val Thr Gln Thr Pro Lys Phe His Val Leu Lys Thr Gly Gln
   1               5                   10                  15
   Ser Met Thr Leu Leu Cys Ala Gln Asp Met Asn His Glu Tyr Met Tyr
               20                  25                  30
   Arg Tyr Arg Gln Asp Pro Gly Lys Gly Leu Arg Leu Ile Tyr Tyr Ser
           35                  40                  45
   Val Ala Ala Ala Leu Thr Asp Lys Gly Glu Val Pro Asn Gly Tyr Asn
       50                  55                  60
   Val Ser Arg Ser Asn Thr Glu Asp Phe Pro Leu Lys Leu Glu Ser Ala
   65                  70                  75                  80
   Ala Pro Ser Gln Thr Ser Val Tyr Phe Cys Ala Ser Ser Tyr Ser Thr
                   85                  90                  95
   Ala Leu Gln Gly Cys Leu Leu Ser Ala His Lys Gly Lys Gly Arg Cys
               100                 105                 110
   Cys Pro Pro Pro Pro Pro Lys Thr Gln Gly Cys Pro Val Gln Arg Ser
           115                 120                 125
   Leu His Gln Glu Pro Trp Asn Pro Glu Trp Pro Gln Val Ala Arg Thr
       130                 135                 140
   Val
   145
<210> 21
   <211> 143
   <212> PRT
   <213> Homo sapiens
<400> 21
   Ala Lys Val Thr Gln Thr Pro Gly His Leu Val Lys Gly Lys Gly Gln
   1               5                   10                  15
   Lys Thr Lys Met Asp Cys Thr Pro Glu Lys Gly His Thr Phe Val Tyr
               20                  25                  30
   Trp Tyr Gln Gln Asn Gln Asn Lys Glu Phe Met Leu Leu Ile Ser Phe
           35                  40                  45
   Gln Asn Glu Gln Val Leu Gln Glu Thr Glu Met His Lys Lys Arg Phe
       50                  55                  60
   Ser Ser Gln Cys Pro Lys Asn Ala Pro Cys Ser Leu Ala Ile Leu Ser
   65                  70                  75                  80
   Ser Glu Pro Gly Asp Thr Ala Leu Tyr Leu Cys Ala Ser Ser Gln Ser
                   85                  90                  95
   Thr Ala Leu Lys Cys Gln Phe Leu Leu Ala His Lys Leu Val Thr Asp
               100                 105                 110
   Pro Ala Gln Glu Ala Gly Asp Val Leu Gly Trp Lys Gly Val Thr Glu
           115                 120                 125
   Asn Asn Trp Ser Gln Leu Lys Pro Gln Cys Asn Leu Thr Gln Gly
       130                 135                 140
<210> 22
   <211> 50
   <212> PRT
   <213> Homo sapiens
<400> 22
   Arg Asp Lys Met Arg Met Gln Arg Ile Lys Val Cys Glu Lys Arg Pro
   1               5                   10                  15
   Ser Ile Asp Leu Cys Ile His His Cys Ser Tyr Phe Gln Lys Cys Glu
               20                  25                  30
   Thr Asn Lys Ile Cys Cys Ser Ala Phe Cys Gly Asn Ile Cys Met Ser
           35                  40                  45
   Ile Leu
       50
<210> 23
   <211> 55
   <212> PRT
   <213> Homo sapiens
<400> 23
   Pro Glu Arg Trp Phe Pro Gly Ser Cys His Val Phe Gly Gln Gly His
   1               5                   10                  15
   Gln Leu Phe His Ile Phe Leu Val Leu Cys Thr Leu Ala Gln Leu Glu
               20                  25                  30
   Ala Val Ala Leu Asp Tyr Glu Ala Arg Arg Pro Ile Tyr Glu Pro Leu
           35                  40                  45
   His Thr His Trp Pro His Asn
       50                  55
<210> 24
   <211> 237
   <212> PRT
   <213> Homo sapiens
<400> 24
   Gln Leu Leu Met Tyr Gln Gln His Thr Ser His Tyr Asp Leu Glu Arg
   1               5                   10                  15
   Lys Gly Gly Tyr Leu Met Leu Ser Phe Ile Asp Phe Cys Pro Phe Ser
               20                  25                  30
   Val Met Arg Leu Arg Ser Leu Pro Ser Pro Gln Arg Tyr Thr Arg Gln
           35                  40                  45
   Glu Arg Tyr Arg Ala Arg Pro Pro Arg Val Leu Glu Arg Ser Gly Phe
       50                  55                  60
   His Asn Glu Asn Ser Leu Ala Ile Tyr Gln Gly Leu Val Tyr Tyr Leu
   65                  70                  75                  80
   Leu Trp Leu His Ser Val Tyr Asp Lys Pro Tyr Ala Asp Pro Val His
                   85                  90                  95
   Asp Pro Thr Trp Arg Trp Trp Ala Asn Asn Lys Gln Asp Gln Asp Tyr
               100                 105                 110
   Tyr Phe Phe Leu Ala Ser Asn Trp Arg Ser Ala Gly Gly Val Ser Ile
           115                 120                 125
   Glu Met Asp Ser Tyr Glu Lys Ile Tyr Asn Leu Glu Ser Ala Tyr Glu
       130                 135                 140
   Leu Pro Glu Arg Ile Phe Leu Asp Lys Gly Thr Glu Tyr Ser Phe Ala
   145                 150                 155                 160
   Ile Phe Leu Ser Ala Gln Gly His Ser Phe Arg Thr Gln Ser Glu Leu
                   165                 170                 175
   Gly Thr Ala Phe Gln Leu His Ser Gln Val Asp Val Gly Val Val Leu
               180                 185                 190
   Ala Asp Pro Gly Cys Ile Glu Ala Ser Val Lys Gln Glu Val Leu Ile
           195                 200                 205
   Asn Arg Asn Ser Val Leu Phe Ser Ile Thr Leu Lys Asp Lys Lys Leu
       210                 215                 220
   Cys Tyr Asp Gln Gly Ile Ser Gly His His Leu Met Glu
   225                 230                 235
<210> 25
   <211> 140
   <212> PRT
   <213> Homo sapiens
<400> 25
   Val Val Glu Glu Leu Lys Leu Ser His Asn Pro Leu Lys Ser Ile Pro
   1               5                   10                  15
   Asp Asn Ala Phe Gln Ser Phe Gly Arg Tyr Leu Glu Thr Leu Trp Leu
               20                  25                  30
   Asp Asn Thr Asn Leu Glu Lys Phe Ser Asp Gly Ala Phe Leu Gly Val
           35                  40                  45
   Thr Thr Leu Lys His Val His Leu Glu Asn Asn Arg Leu Asn Gln Leu
       50                  55                  60
   Pro Ser Asn Phe Pro Phe Asp Ser Leu Glu Thr Leu Ala Leu Thr Asn
   65                  70                  75                  80
   Asn Pro Trp Lys Cys Thr Cys Gln Leu Arg Gly Leu Arg Arg Trp Leu
                   85                  90                  95
   Glu Ala Lys Ala Ser Arg Pro Asp Ala Thr Cys Ala Ser Pro Ala Lys
               100                 105                 110
   Phe Lys Gly Gln His Ile Arg Asp Thr Asp Ala Phe Arg Ser Cys Lys
           115                 120                 125
   Phe Pro Thr Lys Arg Ser Lys Lys Ala Gly Arg His
       130                 135                 140

## Description

### FIELD OF INVENTION

The present invention relates to the field of immunodiagnostic and/or prognostic of liver autoimmune diseases.

### STATE OF THE ART

Autoimmune Liver Diseases (AILD) are chronic and progressive disorders with a poorly understood etiology. The most common AILD are Autoimmune Hepatitis (AIH) and Primary Biliary Cirrhosis (PBC).

Autoimmune Hepatitis (AIH) is a chronic necro-inflammatory disease and one of the most common autoimmune liver diseases AIH has an incidence of 1-2 per 100,000 per year, and a prevalence of 1-10/100,000. As with most of the other autoimmune diseases, it affects women more often than men (80%), with a sex ratio of about 3:1 (female to male) (Czaja AJ. et al., 2010, Gastroenterology; Makol et al., 2011, Hepatitis research and treatment)*.* Histologically it is characterized by: interface hepatitis and plasma cell infiltration; hypergammaglobulinemia is often present; a number of autoantibodies can be detected such as antinuclear antibodies (ANA), anti-Smooth Muscle Antibody (SMA), liver/kidney microsomal antibody (LKM-1), LC1, anti-actin, anti-ASGPR (Bogdanos DP. et al., 2009, Semin Liver Dis)*.* Primary Biliary Cirrhosis (PBC) is a slowly progressing disease causing the destruction of small and medium-size intra-hepatic bile ducts (Selmi C. et al., 2011, Imm Cell Bio). It affects women in 90% of cases. The prevalence is estimated at 0.6-40/100.000. Ursodeoxicholic acid has been shown to improve serum biochemistry, histology and patient's survival (Muratori L. et al., 2010, Dig Liver Dis; Muratori L. et al., 2008, Clin Liver Dis)*.* It is characterized by: anti-mitochondrial antibodies -AMA- (∼90%) and intrahepatic cholestasis (increased alkaline phosphatase -Alk Ph-, normal ultrasonographic -US- scan).

The detection of the AMA autoantibodies is performed routinely by immunofluorescence on fresh multi-organ sections (liver, kidney, stomach) from rodents, but this technique may present many intrinsic problems such as standardization and interpretation of the immuno-morphological patterns (Bogdanos et al., 2008, WJG)*.* To overcome these methodological problems, the International Autoimmune Hepatitis Group established an internationally representative committee to define guidelines and develop procedures and reference standards for more reliable testing (Vergani et al., 2004, Journal of hepatology)*.* In recent years, some AILD target-autoantigens have been identified and characterized (Zachou, K., et al., 2004; J of Autoimm Dis), but little is known on their pathogenetic role, and probably many autoantigens are still unknown. For autoantibodies to have a pathogenetic role, two features have to be met: (i) the autoantigen should be expressed on the target organ and exposed to autoantibodies, (ii) the autoantibodies should have functional activity. Song Q. et al. (2010, J. Proteome Res) described the identification of highly specific biomarkers and their validation for AIH. This study demonstrates that the combination of six autoantigens can be used to diagnose AIH-positive serum samples and that these autoantigens can be effectively used in protein microarray assays, as well as, in traditional ELISA-based assays.

US2009/0023162 discloses the methods for the identification of atypical antineutrophil cytoplasmic antibodies (ANCA), kits suitable for the same and application of said methods to the diagnosis of chronic inflammatory intestinal diseases and autoimmune liver diseases.

RU 2247387 (C1) provides a method involving determination of anti-mitochondrial antibodies, immunoglobulins such as IgA, IgM, IgG, gamma-globulins, anti-gliadin antibodies, and circulating immune complexes for the diagnosis of autoimmune liver injuries in patients with chronic hepatitis.

To date, however, there are no early and precise assays that can be used to identify individuals carrying or at risk of developing AILD. An early diagnosis is clearly important.

Dalekos G. 2002 European J. Int. Medicine, vol. 13, n. 5, pp. 293-303 and Jones D.E. 2000 Journ. Clin. Pathol. Vol. 53, n. 11, pp. 813-821 disclose some autoantigens in AIH and PBC.

### DESCRIPTION OF THE INVENTION

Though the identification of some autoantibodies is within prior art documents, there is still the need to identify novel biomarkers, namely autoantibodies, to diagnose the liver autoimmune disease (AILD) and/or to discriminate between autoimmune and other liver pathologies, and/or to monitor the efficacy of patient treatments of liver autoimmune disease and the disease progression. In the present disclosure a subject with autoimmune liver disease is named "AILD or hepatic autoimmunity patient" and is affected by AIH or PBC.

In the present disclosure a panel of 17 autoantigens was identified in patients with AILD by protein array. In addition, 6 out of the 17 autoantigens were also validated by Dissociation Enhanced Lanthanide FluoroImmunoAssay method (DELFIA^{®}), in patients diagnosed with liver autoimmune diseases, and showed individual sensitivities ranging from 42% to 74%. The combined assessment of these six autoantigens displays a 82%±4% sensitivity and almost 90%±3% specificity.

These six autoantigens represent novel markers of liver autoimmunity, such as AIH and PBC. These markers can display much higher sensitivity and specificity (Vs other diseases such as HCV and HBV) when compared to the benchmark markers (CYP2D6 & ASGPR, as described in the Methods section). Therefore, the autoantigens identified are valuable tools for the development of a new serological assay that is easy to perform and is highly specific for AILD diseases. This assay could significantly contribute to an improvement of AILD diagnosis and to a discrimination between PBC and AIH.

It is therefore an object of the present invention to provide an *in vitro* method of diagnosis or prognosis or evaluation of risk to develop a liver autoimmune disorder belonging to the group of AIH and PBC in a subject, comprising the steps of:
a) contacting a biological sample from the subject with a protein having the amino acid sequence SEQ ID No. 1 under conditions appropriate for binding of autoantibodies, if present in the biological sample, to said protein, and
b) detecting the presence of bound autoantibodies. In the context of the instant disclosure the term "protein" includes:
   i. the whole protein, allelic variants and orthologous thereof;
   ii. any immunological, synthetic or recombinant or proteolytic fragment of a protein having the ability to be recognized by and bound to antibodies directed against the protein;
   iii. any functional equivalent comprised in the group of synthetic or recombinant functional analogs having the ability to be recognized by and bound to antibodies directed against the protein.

In a preferred embodiment, step a) is performed by contacting said biological sample with the protein having the amino acid sequences SEQ ID No. 1 and at least one further protein selected from the group of 16 proteins having the amino acid sequences SEQ ID No. 2, 3, 5, 6, 7, 8, 9, 10, 11, 12, 14, 15, 16, 17, 22, 25, allelic variants, orthologous, immunological fragments or functional equivalents thereof.

In a further embodiment step a) is performed by contacting said biological sample with three proteins having the amino acid sequences SEQ ID No. 1, 11, 17 allelic variants, orthologous, immunological fragments or functional equivalents thereof.

In a further preferred embodiment step a) is performed by contacting said biological sample with four proteins having the amino acid sequences SEQ ID No 1, 10, 11, 17, allelic variants, orthologous, immunological fragments or functional equivalents thereof.

In a further preferred embodiment step a) is performed by contacting said biological sample with six proteins having the amino acid sequences SEQ ID No 1, 6, 8, 10, 11, 17 allelic variants, orthologous, immunological fragments or functional equivalents thereof.

Preferably the biological sample is comprised in the group of blood, serum, plasma, urine, saliva, mucus, or fractions thereof.

Preferably the biological sample is from an adult or from an adolescent. Preferably the detection of said bound autoantibodies is performed by means of binding to specific ligands. More preferably the ligands are conjugated with detecting means.

Also disclosed is a method of monitoring an autoimmune liver disorder after treatment with surgery and/or therapy in a subject with said autoimmune liver disorder, comprising the step of following the modulation of antibodies as disclosed.

In a preferred aspect said proteins or immunological fragments or functional equivalents thereof are displayed on one or more protein microarrays.

It is another object of the invention to provide the use of a protein microarray comprising at least the proteins as defined or immunological fragments or functional equivalents thereof for performing the method according to the invention.

It is another object of the invention to provide the use of a solid support for an immunodiagnostic assay comprising at least the proteins as defined or immunological fragments or functional equivalents thereof for performing the method according to the invention.

Also disclosed is the use of an immunodiagnostic kit comprising the above solid support and detecting means for performing the method according to the invention.

In the present disclosure a subject with autoimmune liver disease is named "AILD or hepatic autoimmunity patient" and is affected by any autoimmune liver disease, as AIH or PBC.

HCV patients are patients diagnosed with hepatitis C and displaying autoreactive antibodies, or patients with HCV infection or patients with hepatitis C and non hepatic autoimmune diseases such as crioglobulinemia or thyroid dysfunctions.

### DETAILED DESCRIPTION OF THE INVENTION

The invention will be now described by means of non limiting examples referring to the following figures.

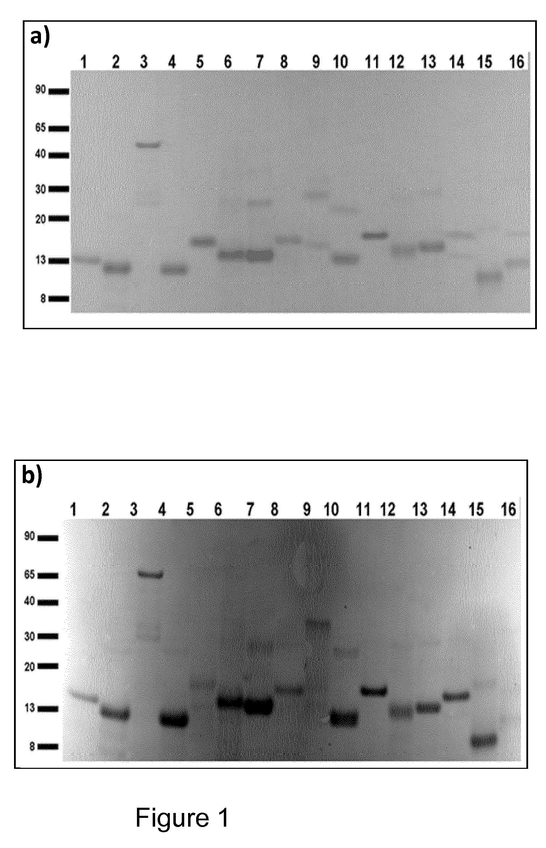

**Figure 1: a)** SDS-PAGE of a representative set of purified recombinant human proteins stained with Coomassie blue; **b)** Western Blot analysis using an anti-histidine (His) tag monoclonal Antibody (mAb); molecular weight markers are shown on the left in kDa.

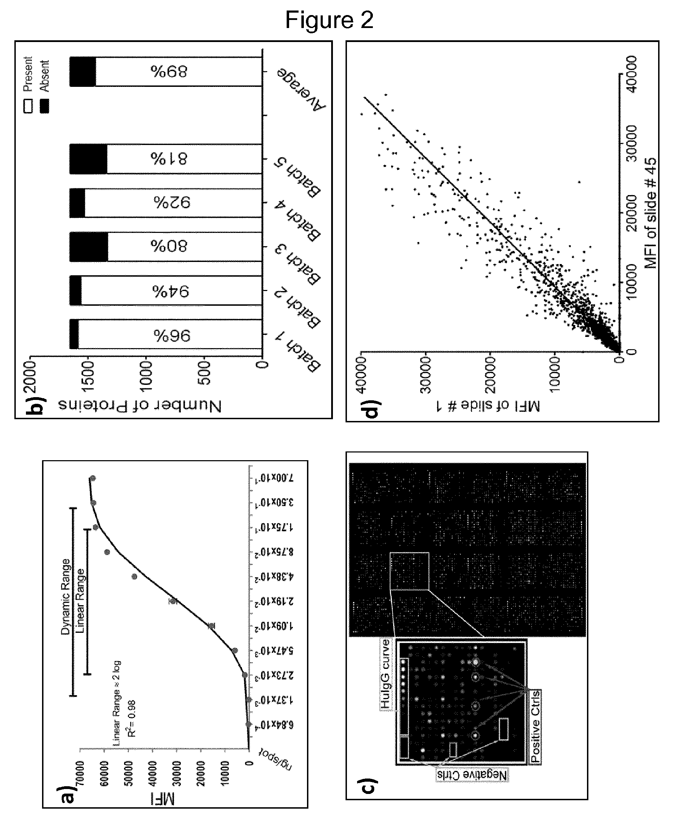

**Figure 2: a)** Plot of the control human IgG curve. The dots correspond to the experimental average signal (Mean Fluorescence Intensity, y-axis) detected for each IgG concentration reported on the x-axis in nanograms per spot (ng/spot), while the continuous line corresponds to the interpolated resulting sigmoid curve. *Dynamic Range:* refers to a range of values that can be measured by a signal (i.e. Mean Fluorescence Intensity). *Linear Range:* is the range of concentration over which the signal intensity is linear, i.e. directly proportional to the spot concentration. **b)** Number of proteins detected with the anti-His mAb. About 90% of proteins were spotted with success on the slides (i.e. about 90 % of the proteins produced signals that were significantly above the background signal). Histograms: Proteins were considered *"Present"* when at least two out of the four replicates gave a signal above the background, otherwise they were considered *"Absent".* **c)** The human protein microarray containing 1626 His-tagged recombinant proteins was probed with an anti-His mAb followed by a secondary α-mouse antibody Alexa-647-labeled. The whole microarray included 24 grids (as the one shown in the enlarged box) each one containing 304 proteins spots and including also positive (such as viral and bacterial proteins) and negative (such buffer or BSA) controls (positive Ctr and negative Ctr frames), as well as IgG calibration curve (HulgG curve) for data normalization. Proteins and controls were always spotted in quadruplicates, while IgG were spotted in eight replicates**.d)** Correlation among spot intensities of two different slides (Slide 1 Vs Slide 45) of the same batch. The scatter plot indicates a positive correlation. The correlation coefficient is 0.9, indicating a high reproducibility of the signals derived from the proteins spotted.

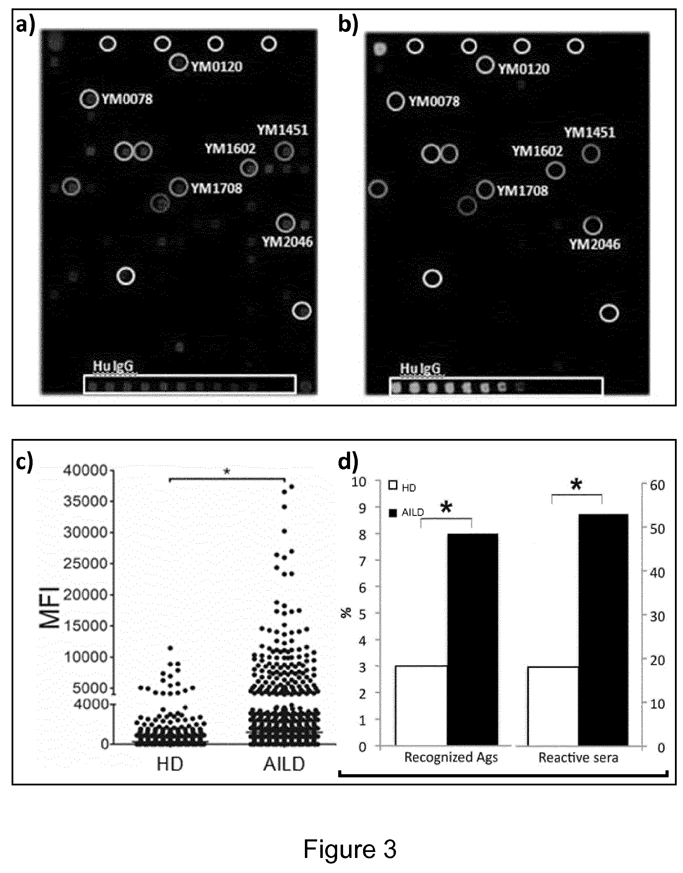

**Figure 3****:** Differences in immunoreactivity between serum samples. Representative image of the autoantigen microarrays incubated with **a)** sera from an AILD patient and **b)** a healthy donor (HD). **c)** Comparison of Mean Fluorescence Intensities (MFIs) of all spotted proteins against two sera populations: Healthy Donor (HD), Autoimmune Liver Diseases (AILD) patients. Each dot represents the MFI of a single protein. A cut-off value ≥ 4.000 was used to score a protein as positive. Asterisks: statistical significance, Student's t-test (pval <0.01). **d)** Percentage of antigens recognized by more than 15% of the sera tested; the threshold was determined on the basis of the average HD recognition (left bars), and the percentage of reactive sera for each population (i.e. sera reacting with more than 3% of the proteins screened; the threshold was determined on the basis of the average HD reactivity) (right bars). Asterisk; statistical significance (χ² test (pval <0.01).

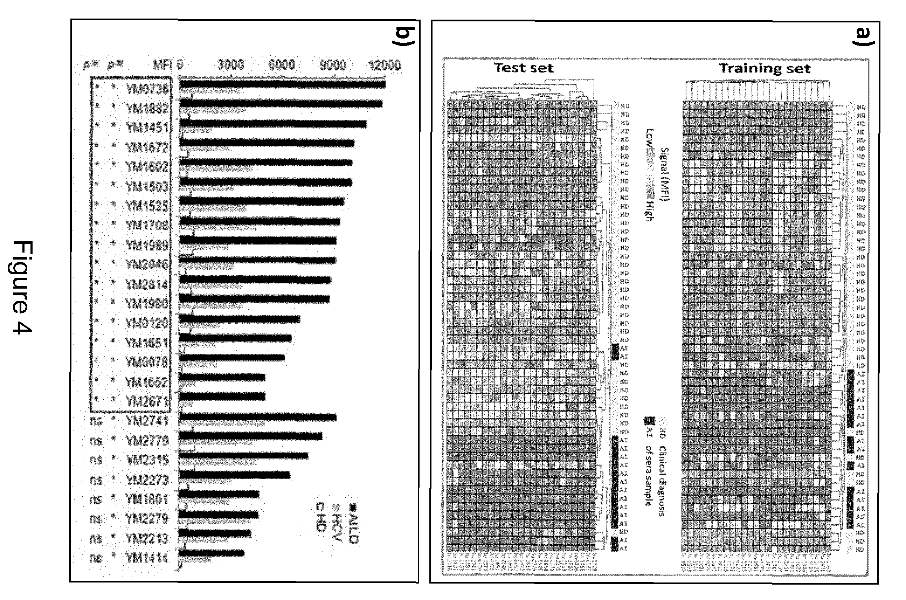

**Figure 4: a)** Hierarchical Clustering of *Training* (upper panel) and *Test set* (lower panel) with regard to the 25 autoantigens selected as specifically recognized by AILD patients on the basis of i) higher recognition frequency and ii) higher mean fluorescence intensities as compared to healthy donors. Sera are represented in columns while proteins are in rows. Red indicates positive immunoreactivity, and blue low or no immunoreactivity; **b)** Bar graph of Mean Fluorescence intensities of the 25 autoantigens selected in Discovery sample set (*Training* and *Test set*). Significant differences were observed between AILD and HCV patients sera for seventeen autoantigens (into the box). Asterisks, p val<0.01 (Student's t-test). P^{(a)}: p-val of AILD *versus* Hepatitis C patients, P^{(b)}: p-val of AILD *versus* Healthy donors.

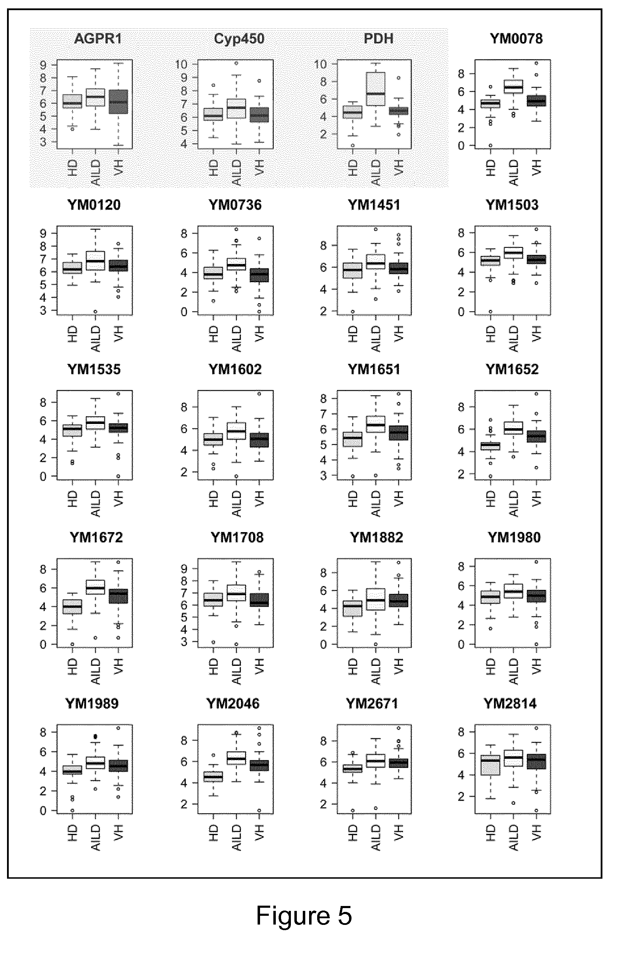

**Figure 5****:** Boxplots of DELFIA results for the 17 autoantigens tested. The signal distributions, after natural logarithm trasformation, are displayed. The boxes define the interquartile range (IQR). The extreme outliers beyond the 1.5 IQR + median are showed as dots. Three known AILD control proteins (AGPR-1, CYP2D6, PDH) (grey box) were also compared. HD: Helathy donors; AILD: autoimmune liver disease, VH: Viral hepatitis.

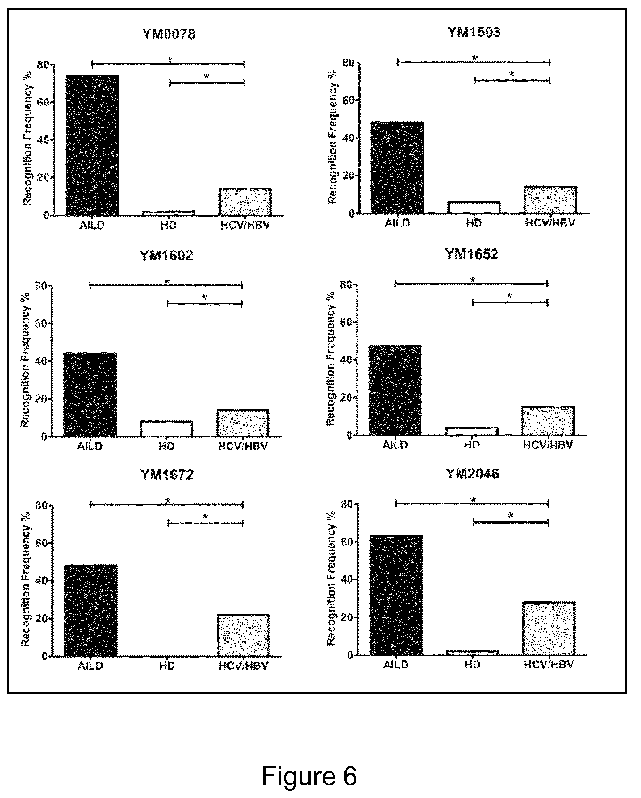

**Figure 6****:** Six proteins are confirmed as AIH autoantigens in DELFIA^{®} assay. Recognition frequency of the best six autoantigens as determined by DELFIA^{®}. Proteins were tested with sera from AILD patients (n. 50 AIH and n. 50 PBC), healthy donors (n. 50) and HBV or HCV viral hepatitis (VH) patients (n. 74). Each sera was tested 3 times in independent experiments. Asterisk: statistical significance (Fisher exact test, pval <0.01).

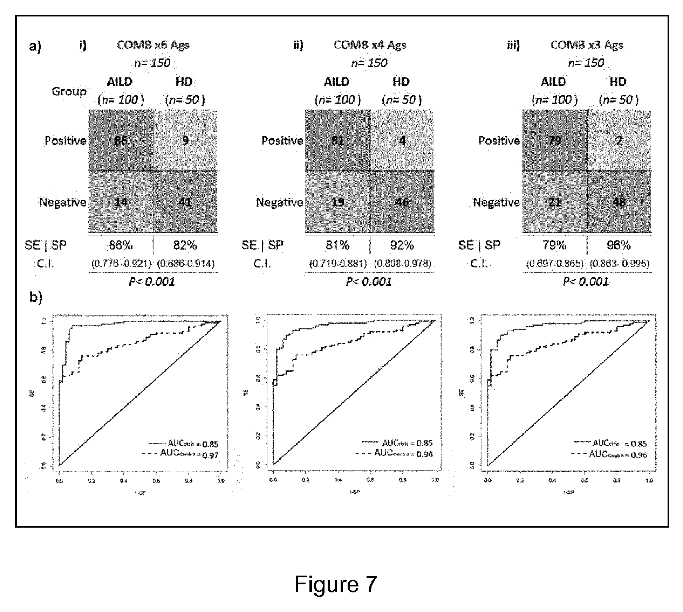

**Figure 7****:** Combination of the six autoantigens identifies AILD patients with high sensitivity and specificity. (a) Numbers in the boxes indicate AIH and HD sera that, in DELFIA^{®} assay, recognize (positive) or do not recognize (negative) combination of six (i), four (ii) or three (iii) of the six autoantigens. Sensitivity (SE) and Specificity (SP) with 95% confidence intervals (C.I.) are indicated in the lower part of the panels. p-values are calculated with χ² tests. (b) Logistic regression models for the six, four and three autoantigens (red curves) and the three known control proteins (AGPR1, CYP2D6, PDH) (black curves) were calculated and represented as ROC curves and corresponding values of Area Under the Curve (AUC).

### Material and Methods

### Serum Samples

Samples used for this study were collected in five different hospitals: i) Policlinico Ospedale Maggiore, Transfusional Unit, Milan; ii) Hepatology Unit, University Hospital, Pisa, Italy; iii) Sant'Orsola-Malpighi University Hospital, Bologna, Italy; iv) Center for Autoimmune Liver Diseases, IRCCS Istituto Clinico Humanitas, Rozzano, Italy; v) Center for Systemic Manifestations of Hepatitis Viruses (MaSVE), University of Firenze, Italy. For the discovery phase, 218 sera were used (15 AIH, 15 PBC 78 HD, 110 HCV), while for the validation phase 224 sera were used (50 AIH, 50 PBC, 50 HD, 50 HCV, 24 HBV). Table 1 reports the clinical characteristics and ages of the patients and donors enrolled in this invention, for the microarray analysis (Example 2-3).

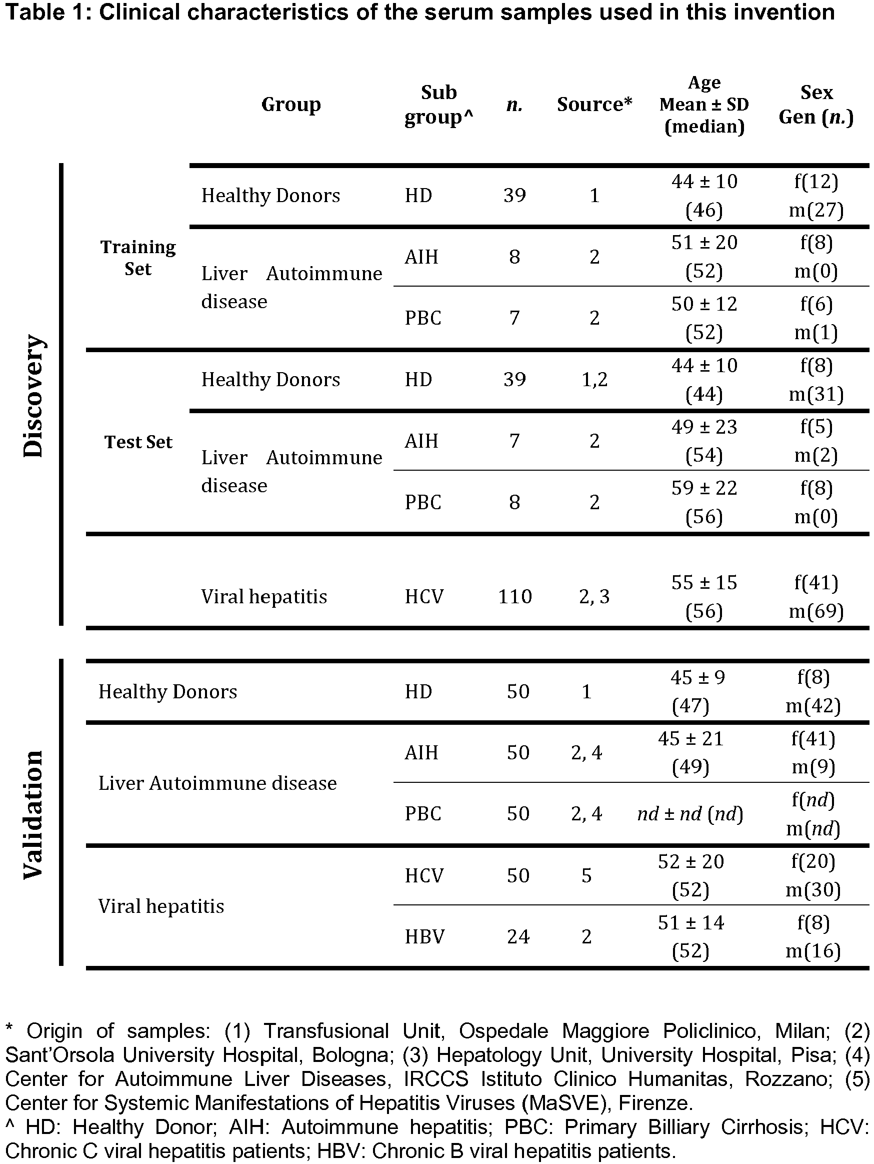

### Human proteins: selection, expression and purification

Genes whose translated products carry a secretion signal peptide or at least one transmembrane domain were selected, cloned and expressed in a high through-put system as histidine-tagged products as described (Grifantini R. et al., 2011, Journal of proteomics). 1626 full-length proteins or protein domains were expressed in *E. coli* and purified from the bacterial insoluble fraction by Immobilized metal ion affinity chromatography (IMAC, GE).

Human, viral or bacterial proteins were used as biological or technical controls in the microarray. In particular genes encoding for Core protein and Non-structural proteins NS3 (from HCV genotype 1), NS3-4a (from HCV genotype 2) NS5b (from HCV genotype 1), Tetanus toxin and H1N1 were subcloned in *E. coli* strain DH5α and expressed in BL21(DE3), respectively. Bovine Serum Albumin (BSA), Human Serum Albumin, Human Glutathione-S-Transferase and Protein A from *Staphylococcus aureus* were purchased from Sigma.

For DELFIA^{®} experiments, Pyruvate DeHydrogenase (PDH) protein was purchased by Sigma, while genes encoding Cytochrome P450 2D6 (CYP2D6) and asialoglycoprotein receptor 1 (ASGR-1) from Ultimate™ Human ORF Clones were purchased by Invitrogen and were subcloned in *E. coli* strain DH5α and expressed in BL21(DE3), respectively. All the corresponding proteins were purified by affinity chromatography on IMAC resin.

### Protein quality control

Purified recombinant proteins (10-15 µg total protein), obtained as described above, were stored at 4°C and analyzed by SDS-PAGE (Criterion PAGE system Bio-Rad) followed by Coomassie Blue staining of the gel immediately before spotting them, to assess their integrity, and purity level. Proteins showing purity levels >70% (ChemiDoc™ XRS, Quantity One^{®} software; Bio-Rad) were used for protein array preparation.

For Western blot analysis, aliquots (0.5 µg) of the proteins were resolved on 4-12% pre-cast SDS-PAGE gradient Tricine gels under reducing conditions, and electroblotted onto nitrocellulose membranes (Bio-Rad), according to the manufacturer's instructions. The membranes were blocked with 5% non-fat milk in 1x PBS plus 0.1% Tween 20 (TPBS) for 1 h at room temperature, incubated with the α-His mAb (GE-Healthcare) diluted 1:1000 in 3% non-fat milk in TPBS 0.1% for 1 h at room temperature, and washed three times in TPBS 0.1% (Fig. 1). The secondary HRP-conjugated antibody (α-mouse immunoglobulin/HRP, GE-Healthcare) was diluted 1:1000 in 3 % non-fat milk in TPBS-0.1 % and incubated for 1 h at room temperature. The proteins were visualized by enhanced chemiluminescence (Super Signal West Pico Chemiluminescence Substrate Thermo Scientific, USA) according to the manufacturer's specifications. Chemiluminescence was detected with an LAS-3000 (Fujifilm, USA).

### Protein microarray printing

Protein MicroArrays were generated by spotting the 1626 affinity-purified recombinant proteins (0.5 mg/ml, in 6M Urea) in 4 replicates on nitrocellulose-coated slides (FAST slides, GE-Healthcare) using Stealth SMP3^{®} spotting pins (TeleChem International, Sunnyvale, California) and a Microgrid II microarray contact printer (Biorobotics), resulting in spots of approximately 130 µm in diameter. As experimental positive control, to assess the sensitivity and reproducibility of the arrays and for signal normalization, a curve of human IgG(s) at 11 different concentrations (solutions from 0.001 to 1 mg/ml) was spotted on the arrays in 8 replicates (in 6M Urea) and detected with Alexa-647 conjugated α-Human IgG secondary antibody (Invitrogen). As negative controls the spotting buffer alone, was printed and used to assess possible non-specific signals due to cross contamination.

A quality control of the spotting procedure was performed on 10% of randomly chosen slides, by confirming the presence of the total immobilized proteins using the α-His mAb, followed by detection using a Alexa-647 conjugated α-Human IgG secondary antibody (Fig. 2). The spotted microarrays were allowed to remain at room temperature for 1 h before storage at 4 °C until use.

### Incubation and scanning of protein microarray

Incubation was automatically performed with a TECAN Hybridization Station (HS 4800™ Pro; TECAN, Salzburg, Austria). The microarray slides were prewashed 3 min in TPBS 0.1% Tween 20, and saturated with Blocklt™ Microarray Blocking Buffer (Arrayit Corporation) for 45 min at 25°C under mild agitation. After injection of 105 µl of individual serum (diluted 1:300 in Blocking Buffer plus 0.1% Tween 20), incubation was performed at 25°C for 45 min with low agitation. The microarrays were washed at 25°C in TPBS for three cycles of 1 min wash time and 30 sec soak time.

Afterwards the microarray slides were incubated at 25°C for 1 with Alexa-647 conjugated α-human IgG (Invitrogen) diluted at 1:800 in Blocking Buffer in the dark. The microarrays were again washed at 25°C in TPBS for two cycles of 1 min wash time and 30 sec soak time, in PBS for two cycles of 1 min: 30 sec and finally in milliQ sterile water for one cycle of 15 sec.

The microarray slides were finally dried at 30°C under nitrogen for 2 min, and scanned using a ScanArray Gx PLUS (PerkinElmer, Bridgeport Avenue Shelton, USA). 16-bit images were generated with ScanArrayTM software at 10µm per pixel resolution and processed using ImaGene 8.0 software (Biodiscovery Inc, CA, USA). Laser of 635 nm was used to excite Alexa-647 dye. The fluorescence intensity of each spot was measured, signal-to-local-background ratios were calculated by ImaGene, and spot morphology and deviation from the expected spot position were considered using the default ImaGene settings.

### Data analysis

For each sample, the Mean Fluorescence Intensity (MFI) of replicated spots was determined, after subtraction of the background value for each spot, and subsequently normalized on the basis of the human IgG curve to allow comparison of data from different set of experiments (Bombaci M. et al., 2009, PLoS One). Briefly, the MFIs values of IgG, spotted at different concentrations, were best fitted by a sigmoid curve, using a maximum likelihood estimator (Harris JW et al., 1998, Handbook of Mathematics and Computational Science). The experimental average IgG curve of each slide was adjusted on the reference sigmoid IgG curve, and the background-subtracted MFI values of each protein were normalized accordingly. On the basis of these results, a normalized MFI value of 4.000 was chosen as the lowest signal threshold for scoring a protein as positively recognized by human sera. For each protein, a Coefficient of Variation (CV%), was calculated on the four replicate spots, for intra-assay reproducibility (Bombaci et al., 2009, PLoS One).

Recognition Frequency was defined as the percentage of sera reacting with a particular antigen in protein array with a MFI ≥ 4.000, and it was calculated for each group of sera. TIGR Multiexperiment Viewer (version MeV4.5) software (Saeed, A.I., et al., 2006, Methods in enzymology) was used to perform an unsupervised bi-dimensional hierarchical clustering.

### Dissociation-Enhanced Lanthanide Fluorescence ImmunoAssay DELFIA^{®} assays

The DELFIA^{®} assay is a time-resolved fluorescence method that can be used to study antibody binding to solid-phase proteins or peptides. The purified recombinant proteins were used at a concentration of 20 µg per milliliter (Frulloni L. et al., 2009, N Engl J Med) in 6 M urea to coat DELFIA^{®} plates (PerkinElmer). Plates were then blocked for 1 hour at 37°C with a blocking reagent (PerkinElmer). The blocking buffer was than discarded, and the serum samples were diluted in a 1:300 solution in phosphate buffered saline plus 1% bovine serum albumin (Sigma), plus 0.1 %Tween 20 (Sigma) and incubated on the plates 1 hour at 37°C. Plates were then washed 5 times with washing buffer (PerkinElmer). Bound antibodies were detected with europium-labeled α-human IgG serum (1:500 in diluting buffer, PerkinElmer), incubated 30 min at RT in the dark. The wells were again washed in the same washing buffer. After a 10 min incubation at RT, the plates were read on a Infinite F200 PRO instrument (Tecan). Fluorescence intensity values higher than the mean of buffer plus 3 standard deviations were considered to be positive.

### Statistical analysis

Results of Protein Microarray and DELFIA^{®} experiments from sera of patients and healthy donors were compared using the two-tailed χ² test, the Student's t-test or the Fisher's exact tests. The ANOVA test was used for all the others analyses. The Benjamini-Hochberg correction for multiple testing was used for the analysis of microarray data. Statistical analysis was carried out with the use of GraphPad Prism 5 software, version 5.01. To evaluate the performance of autoantigens combinations in discriminating AILD patients from Healthy donors or HCV patients, logistic regression analysis was performed with R. We the signals of respectively 6, 4 or 3 selected autoantigens we created logistic regression models. The probabilities were calculated as follows: p= exp ((Σ-(bᵢxᵢ)+ c)/(1 +Σ(bᵢxᵢ)+ c), where p is the probability of each case, i= 1 to n; b is the regression coefficient of a given autoantigen, x is signal intensity and c is a constant generated by the model. ROCR package was used to obtain the ROC curves of the models and the Area Under Curve (AUC) values (Sing T. et al. 2005, Bioinformatics)*.*

### EXAMPLES

### Example 1- Design and Development of a Human Protein Microarray

To study the serological profile of patients diagnosed with autoimmune liver diseases versus a panel of self proteins, the authors developed a protein array by printing 1626 human recombinant (see details in *Materials and Methods* section) products that corresponded to 1371 distinct human proteins distributed as shown in Table 2.

**Table 2: Predicted subcellular localization of the human recombinant proteins used for the microarray construction (Grifantini et al., 2011).**

| **Compartment** | **% of proteins** |
|---|---|
| Cell membrane | 46 |
| Secreted | 23 |
| Intracell. Membrane | 11 |
| Cytoplasm | 11 |
| Mito. Membrane | 4 |
| Nucleus | 4 |
| Mithocondrion | 1 |

Briefly, 1329 of the 1371 the proteins were first selected through a bioinformatic analysis of the whole human genome as translated sequences carrying i) signal peptides, ii) at least one transmembrane domain, iii) having unknown biological function. Fortytwo of the 1371 proteins had a well known immunological function, CD number assigned and were all surface exposed. The majority of printed human proteins were expressed as N-terminal His-tag fusions while 48 proteins where expressed as double tagged fusions, with an N-terminal glutathione S-transferase (GST) and with C-terminal Histidines. Proteins obtained after affinity purification from the bacterial insoluble fraction showed purity levels >70%, as estimated by densitometric scan of SDS-PAGE gels (see *Materials and Methods)* (Figure 1a). Moreover, we performed western blot analysis on random protein samples with the anti-His mAb. About 80% of the probed proteins gave a positive signal. In addition for almost 90% of the latter proteins, the molecular weights of the bands detected by western blot corresponded to those observed by SDS-PAGE (Figure 1 b).

Protein arrays were prepared by printing onto nitrocellulose-covered glass slides four replicates of each protein. In addition, we included in the array, several biological and technical controls including human, viral and bacterial proteins (*Materials and Methods*)*.* Replicates were randomly distributed to get optimal signal reproducibility. Moreover. eleven different amounts of human IgGs at a known concentration (from 8.24x10⁻⁴ to 7x10⁻¹ ng of immobilized protein/spot) were printed also on the array in eight replicates(Figure 2a). The quality of the immobilized proteins on the arrays were determined by probing 10% of the slides with an anti-His mAb, and 89% of the proteins produced signals that were significantly above the background (Figure 2b). The final protein array design consisted of 24 grids each of 304 spots, for a total of 7296 spots (Figure 2c). The correlation among spot intensities of two different slides of the same batch indicates a high reproducibility of the signals derived from the proteins spotted (Figure 2d).

### Example 2- Patients affected by autoimmune liver diseases show higher auto-immunoreactivity as compared to healthy donors

In an attempt to determine a panel of autoantigens differentially recognized by patients sera with AILD compared to healthy individuals (HD), the protein microarrays were probed with a sample set (defined as *Training set* as indicated in Table 1 in *Materials and Methods)* comprising 15 sera patients with AILD, and 39 sera from healthy donors. The clinical characteristics of each group of sera are summarized in Table 1.

Sera reactivity was evaluated by detecting total IgG bound to each protein spot using Alexa-647 conjugated α-human IgG and measuring the fluorescence intensity (FI) values for each protein. To compare data from different experiments, we used a normalization method, as previously described (Bombaci M et al., 2009, PLoS One)*.* Briefly, the experimental average IgG curve of each slide was adjusted on a reference sigmoid IgG curve, and the background-subtracted mean fluorescence intensities (MFIs) values of each protein were normalized accordingly. On the basis of these results, a normalized-MFI value of 4000 was chosen as the lowest signal threshold for scoring a protein as positively recognized by human sera.

First of all, total reactivity of patients against healthy donors sera was evaluated. Representative images of a zoomed grid of the microarray probed with sera derived from healthy donors and AILD patients are shown in Figure 3a and b. As depicted in Figure 3c, the strongest autoreactivity was observed against all the proteins present on the arrays in patients sera with AILD, but not in the control sera (HD group).

Significant divergences between patients and healthy donors were also observed in terms of the recognition frequencies, indicating differential protein-specific IgG levels Figure 3d: in details, the percentage of proteins recognized by more than 15% of the tested sera with MFI>4000 was about 8% (132 proteins) in the case of both patients groups but only 3% (41 proteins) in the case of healthy donors (left). On the other hand, 53% of patients with AILD contain antibodies that react intensively with at least 3% of the proteins on the arrays, whereas only 18% of 39 controls had such antibodies (rigth panel). In both cases the differences observed between the reactivity of patients and healthy donors were statistically significant (chisquare test, p val <0.01).

### Example 3- Selection and identification of specifically recognized autoantigens profile

Having previously established that patients with AILD of *Training set* displayed an increased autoreactivity when compared to healthy donors, the authors selected the autoantigens specifically recognized by those patients. Following normalization, individual autoantigens from protein microarray were ranked according to (i) the recognition frequency and (ii) the mean fluorescence intensity of AILD patients as compared to the healthy donors. To be considered of potential interest, the antigen-specific responses had to occur with a significantly higher signal intensity in patients sera than in healthy donors sera (T test's p val<0.01). In particular, the proteins should be recognized in less than 10% of the healthy donors sera and in at least 25% of sera from patients groups (Fisher test's pval <0.01). By using this approach, the authors identified 25 distinct proteins (Table 3) showing a higher immunoreactivity with AILD patients .compared to controls sera.

**Table 3: Brief description of the human sequences listed**

| **SEQ ID NO** | **Prot_ID** | **Accession Protein** | **Amino acid sequence** |
|---|---|---|---|
| NO: 1 | YM0078 | *ENSP00000379111* | |
| NO: 2 | YM0120 | *ENSP00000386923* | |
| NO: 3 | YM0736 | *ENSP00000350556* | |
| NO: 4 | YM1414 | *ENSP00000404259* | |
| NO: 5 | YM1451 | *ENSP00000343084* | |
| NO: 6 | YM1503 | *ENSP00000375259* | |
| NO: 7 | YM1535 | *ENSP00000288466* | |
| NO: 8 | YM1602 | *XP_934154* | |
| NO: 9 | YM1651 | *ENSP00000343493* | |
| NO: 10 | YM1652 | *ENSP00000413076* | |
| NO: 11 | YM1672 | *ENSP00000315731* | |
| NO: 12 | YM1708 | *ENSP00000392824* | |
| NO: 13 | YM1801 | *ENSP00000367965* | |
| NO: 14 | YM1882 | *ENSP00000395006* | |
| NO: 15 | YM1980 | *ENSP00000362968* | |
| NO: 16 | YM 1989 | *ENSP00000415977* | |
| NO: 17 | YM2046 | *ENSP00000360191* | |
| NO: 18 | YM2213 | *ENSP00000414589* | |
| NO: 19 | YM2273 | *ENSP00000374868* | |
| NO: 20 | YM2279 | *ENSP00000374896* | |
| NO: 21 | YM2315 | *ENSP00000374919* | |
| NO: 22 | YM2671 | *ENSP00000327628* | |
| NO: 23 | YM2741 | *ENSP00000363414* | |
| NO: 24 | YM2779 | *ENSP00000395093* | |
| NO: 25 | YM2814 | *ENSP00000258969* | |

In order to confirm the above results, a different set of proteins spotted in the microarray was used. This microarray now included all proteins identified as more reactive in the training set including the 24 proteins as indicated above (Table 4).

**Table 4: Proteins printed on the focused microarray.**

| # | **Accession Protein** | **Description** | **Protein Array_Id** |
|---|---|---|---|
| 1 | *ENSP00000292144* | *CD3g molecule*, *gamma (CD3-TCR complex)* | YM0066 |
| 2 | *ENSP00000418364* | *CD80 molecule* | YM0071 |
| 3 | *ENSP00000377248* | *CD86 molecule* | YM0072 |
| 4 | *ENSP00000228434* | *CD69 molecule* | YM0073 |
| 5 | *ENSP00000216223* | *interleukin 2 receptor, beta* | YM0076 |
| 6 | *ENSP00000379111* | *interleukin 4 receptor* | YM0078 |
| 7 | *ENSP00000345501* | *integrin, beta 7* | YM0079 |
| 8 | *ENSP00000390133* | *transferrin receptor (p90, CD71)* | YM0083 |
| 9 | *ENSP00000262817* | *Transmembrane protein 59-like Precursor* | YM0099 |
| 10 | *ENSP00000361001* | *EF-hand domain-containing protein KIAA0494* | YM0100 |
| 11 | *ENSP00000363041* | *CDGSH iron sulfur domain-containing protein 1* | YM0107 |
| 12 | *ENSP00000296955* | *Discoidin, CUB and LCCL domain-containing protein 1 Precursor* | YM0117 |
| 13 | *ENSP00000386923* | *Lysozyme g-like protein 1 Precursor* | YM0120 |
| 14 | *ENSP00000302046* | *Ribonuclease K6 Precursor* | YM0125 |
| 15 | *ENSP00000317385* | *GLIPR1-like protein 2* | YM0724 |
| 16 | *ENSP00000334044* | *Ubiquitin-like protein 4B* | YM0728 |
| 17 | *ENSP00000350556* | *Uncharacterized protein C19orf47* | YM0736 |
| 18 | *ENSP00000312599* | *Transmembrane protein 70, mitochondrial Precursor* | YM0757 |
| 19 | *ENSP00000334308* | *Nesprin-3* | YM0838 |
| 20 | *ENSP00000258436* | *Major facilitator superfamily domain-containing protein 9* | YM0872 |
| 21 | *ENSP00000357480* | *Nogo-B receptor Precursor* | YM0887 |
| 22 | *ENSP00000412308* | *Uncharacterized protein C18orf19* | YM0908 |
| 23 | *ENSP00000364502* | *Protein FAM70B* | YM0972 |
| 24 | *ENSP00000294258* | *Zinc finger protein-like 1* | YM1020 |
| 25 | *ENSP00000262262* | *CD33 molecule* | YM1046 |
| 26 | *ENSP00000364621* | *Sushi domain-containing protein 3* | YM1073 |
| 27 | *ENSP00000266943* | *Solute carrier family 46 member 3 Precursor* | YM1077 |
| 28 | *ENSP00000292301* | *chemokine (C-C motif) receptor 2* | YM1099 |
| 29 | *ENSP00000317300* | *Lysophosphatidylcholine acyltransferase 4* | YM1129 |
| 30 | *ENSP00000326267* | *EF-hand domain-containing protein C14orf143* | YM1131 |
| 31 | *ENSP00000356048* | *Synaptotagmin-like protein 3* | YM1134 |
| 32 | *ENSP00000342493* | *Regulator of microtubule dynamics protein 3* | YM1183 |
| 33 | *ENSP00000352601* | *Low-density lipoprotein receptor-related protein 10 Precursor* | YM1204 |
| 34 | *ENSP00000347152* | *Attractin-like protein 1 Precursor* | YM1208 |
| 35 | *ENSP00000416040* | *UPF0606 protein KIAA1549* | YM1214 |
| 36 | *ENSP00000248668* | *Leucine-rich repeat and fibronectin type III domain- containing protein 1 Precursor* | YM1217 |
| 37 | *ENSP00000357631* | *Transcription elongation regulator 1-like protein* | YM1340 |
| 38 | *ENSP00000311657* | *GRAM domain-containing protein 2* | YM1342 |
| 39 | *ENSP00000360822* | *Potassium channel subfamily T member 1* | YM1343 |
| 40 | *ENSP00000418985* | *Solute carrier family 22 member 23* | YM1351 |
| 41 | *ENSP00000311307* | *Uncharacterized protein C4orf26 Precursor* | YM1401 |
| 42 | *ENSP00000404259* | *EGF-like domain-containing protein 8 Precursor* | YM1414 |
| 43 | *ENSP00000384553* | *Zinc*/*RING finger protein 3 Precursor* | YM1423 |
| 44 | *ENSP00000343084* | *Putative uncharacterized protein* | YM1451 |
| 45 | *ENSP00000374125* | *Sialic acid-binding Ig-like lectin 15 Precursor* | YM1477 |
| 46 | *ENSP00000375259* | *Putative uncharacterized protein DKFZp667F0711 Fragment* | YM1503 |
| 47 | *ENSP00000263569* | *Platelet receptor Gi24 Precursor* | YM1526 |
| 48 | *ENSP00000247618* | *Kinesin light chain 1* | YM1529 |
| 49 | *ENSP00000368502* | *Secretoglobin-like protein Precursor* | YM1534 |
| 50 | *ENSP00000288466* | *zinc finger protein 618* | YM1535 |
| 51 | *ENSP00000359571* | *Uncharacterized protein CXorf66 Precursor* | YM1536 |
| 52 | *ENSP00000315554* | *Spermatid maturation protein 1* | YM1537 |
| 53 | *ENSP00000415978* | *Protein FAM74A1*/*A2* | YM1542 |
| 54 | *ENSP00000325525* | *similar to Killer cell immunoglobulin-like receptor 3DL2 precursor (MHC class I NK cell receptor) (Natural killer associated transcript 4) (NKAT-4) (p70 natural killer cell receptor clone CL-5)* | YM1546 |
| 55 | *ENSP00000406884* | *HCG2023280cDNA FLJ30064 fis, clone ADRGL2000323 ;* | YM1587 |
| 56 | *NP 001034884* | *Putative uncharacterized protein* | YM1593 |
| 57 | *ENSP00000374867* | *hypothetical protein LOC648852* | YM1594 |
| 58 | *ENSP00000397690* | *Putative uncharacterized protein UNQ6190*/*PR020217 Precursor* | YM1599 |
| 59 | *XP_934154* | *Putative uncharacterized protein* | YM1602 |
| 60 | *ENSP00000343493* | *Uncharacterized protein C17orf99 Precursor* | YM1651 |
| 61 | *ENSP00000413076* | *Uncharacterized protein C17orf99 Precursor* | YM1652 |
| 62 | *ENSP00000368396* | *UPF0631 protein HSD24* | YM1668 |
| 63 | *ENSP00000328061* | *Uncharacterized protein C17ort74* | YM1671 |
| 64 | *ENSP00000315731* | *Meteorin-like protein Precursor* | YM1672 |
| 65 | *ENSP00000331466* | *Transmembrane protein 95 Precursor* | YM1674 |
| 66 | *ENSP00000331466* | *Transmembrane protein 95 Precursor* | YM1675 |
| 67 | *ENSP00000304670* | *RING finger and transmembrane domain-containing protein 1* | YM1684 |
| 68 | *ENSP00000303437* | *t-SNARE domain-containing protein 1* | YM1693 |
| 69 | *ENSP00000392824* | *LP5624* | YM1708 |
| 70 | *ENSP00000346747* | *Putative uncharacterized protein* | YM1720 |
| 71 | *ENSP00000262424* | *Cysteine-rich secretory protein LCCL domain-containing 2 Precursor* | YM1770 |
| 72 | *ENSP00000360577* | *Enoyl-CoA hydratase domain-containing protein 2, mitochondrial Precursor* | YM1778 |
| 73 | *ENSP00000367965* | *Ankyrin repeat domain-containing protein 43 Precursor* | YM1801 |
| 74 | *ENSP00000354240* | *RPE-spondin Precursor* | YM1810 |
| 75 | *OTTHUMP00000028326* | *Putative uncharacterized protein* | YM1824 |
| 76 | *ENSP00000395006* | *Carboxypeptidase-like protein X2 Precursor* | YM1882 |
| 77 | *ENSP00000298966* | *UPF0443 protein C11orf75* | YM1909 |
| 78 | *ENSP00000350961* | *Transmembrane protein C9orf123* | YM1914 |
| 79 | *ENSP00000378605* | *DnaJ homolog subfamily C member 30* | YM1925 |
| 80 | *OTTHUMP00000028097* | *Putative uncharacterized protein* | YM1957 |
| 81 | *ENSP00000368662* | *Uroplakin-3-like protein Precursor* | YM1960 |
| 82 | *ENSP00000405827* | *Putative uncharacterized protein* | YM1971 |
| 83 | *ENSP00000362968* | *Colipase-like protein C6orf127 Precursor* | YM1980 |
| 84 | *ENSP00000409535* | *C-type lectin domain family 18 member B Precursor* | YM1985 |
| 85 | *ENSP00000415977* | *Chondroadherin-like protein Precursor* | YM1989 |
| 86 | *ENSP00000412448* | | YM2013 |
| 87 | *ENSP00000414449* | | YM2036 |
| 88 | *ENSP00000360191* | *Protein APCDD1-like Precursor* | YM2046 |
| 89 | *ENSP00000321517* | *Putative uncharacterized protein* | YM2054 |
| 90 | *ENSP00000339578* | *Seven transmembrane helix receptor* | YM2071 |
| 91 | *ENSP00000397696* | *HSAL5836* | YM2094 |
| 92 | *ENSP00000366415* | *Putative uncharacterized protein* | YM2100 |
| 93 | *ENSP00000345107* | *Putative uncharacterized protein* | YM2110 |
| 94 | *ENSP00000344029* | *Putative uncharacterized protein UNQ9165*/*PR028630 Precursor* | YM2111 |
| 95 | *ENSP00000409458* | *Putative uncharacterized protein* | YM2136 |
| 96 | *ENSP00000398103* | *Putative uncharacterized protein UNQ6493*/*PR021345* | YM2140 |
| 97 | *ENSP00000372305* | *Putative uncharacterized protein* | YM2144 |
| 98 | *ENSP00000411889* | *Putative uncharacterized protein UNQ6490*/*PR021339 Precursor* | YM2151 |
| 99 | *ENSP00000349132* | *Protein FAM75A3* | YM2168 |
| 100 | *ENSP00000406884* | *HCG2023280cDNA FLJ30064 fis, clone ADRGL2000323 ;* | YM2202 |
| 101 | *ENSP00000414589* | *VGSA 5840* | YM2213 |
| 102 | *ENSP00000389279* | *AHPA9419* | YM2214 |
| 103 | *ENSP00000358798* | *Calcium homeostasis modulator protein 3* | YM2223 |
| 104 | *ENSP00000374897* | | YM2237 |
| 105 | *ENSP00000373765* | *monooxygenase, DBH-like 2 pseudogene (MOXD2)* | YM2250 |
| 106 | *ENSP00000374869* | | YM2251 |
| 107 | *ENSP00000304930* | *Sclerostin domain-containing protein 1 Precursor* | YM2255 |
| 108 | *ENSP00000409076* | *Uncharacterized protein KIAA1644 Precursor* | YM2271 |
| 109 | *ENSP00000374868* | *Putative uncharacterized protein ENSP00000374868* | YM2273 |
| 110 | *ENSP00000374880* | | YM2276 |
| 111 | *ENSP00000374896* | *Putative uncharacterized protein ENSP00000374896* | YM2279 |
| 112 | *ENSP00000400516* | *Hematopoietic cell signal transducer Precursor* | YM2284 |
| 113 | *ENSP00000382000* | *CMT1A duplicated region transcript 15 protein-like protein* | YM2286 |
| 114 | *ENSP00000311857* | | YM2289 |
| 115 | *ENSP00000331418* | | YM2297 |
| 116 | *ENSP00000374919* | *Putative uncharacterized protein ENSP00000374919* | YM2315 |
| 117 | *OTTHUMP00000076641* | *Putative uncharacterized protein* | YM2322 |
| 118 | *OTTHUMP00000076959* | *Putative uncharacterized protein* | YM2326 |
| 119 | *ENSP00000222033* | *Zinc*/*RING finger protein 4 Precursor* | YM2335 |
| 120 | *ENSP00000251473* | *Lipid phosphate phosphatase-related protein type 2* | YM2355 |
| 121 | *ENSP00000291934* | *Transmembrane protein 190 Precursor* | YM2388 |
| 122 | *ENSP00000393015* | *Serine protease 23 Precursor* | YM2441 |
| 123 | *ENSP00000414523* | *Leucine-rich repeat transmembrane neuronal protein 1 Precursor* | YM2448 |
| 124 | *ENSP00000363345* | *Thymic stromal cotransporter homolog* | YM2453 |
| 125 | *ENSP00000307164* | *Prostate and testis expressed protein 1 Precursor* | YM2511 |
| 126 | *ENSP00000355243* | *Protocadherin-7 Precursor* | YM2570 |
| 127 | *ENSP00000264661* | *Potassium voltage-gated channel subfamily H member 4* | YM2576 |
| 128 | *ENSP00000266646* | *Inhibin beta E chain Precursor* | YM2613 |
| 129 | *ENSP00000344847* | *A disintegrin and metalloproteinase with thrombospondin motifs 12 Precursor* | YM2616 |
| 130 | *ENSP00000298690* | *Ribonuclease 7 Precursor* | YM2626 |
| 131 | *ENSP00000321345* | *interleukin 23 receptor* | YM2646 |
| 132 | *ENSP00000361735* | *INAP four-disulfide core domain protein 8 Precursor* | YM2665 |
| 133 | *ENSP00000327628* | *Protein INFDC108 Precursor* | YM2671 |
| 134 | *ENSP00000259216* | *Cryptic protein Precursor* | YM2707 |
| 135 | *ENSP00000349131* | *R-spondin-3 Precursor* | YM2726 |
| 136 | *ENSP00000347451* | *Leucine-rich repeat and immunoglobulin-like domain- containing nogo receptor-interacting protein 1 Precursor* | YM2728 |
| 137 | *ENSP00000363414* | *Membrane progestin receptor alpha* | YM2741 |
| 138 | *ENSP00000385766* | *Leucine-rich repeat-containing protein 32 Precursor* | YM2767 |
| 139 | *ENSP00000395093* | *Uncharacterized protein C19orf15 Precursor* | YM2779 |
| 140 | *ENSP00000258969* | *Chondroadherin Precursor* | YM2814 |
| 141 | *ENSP00000272134* | *left-right determination factor 1* | YM2826 |
| 142 | *ENSP00000215885* | *Group* 3 *secretory phospholipase A2 Precursor* | YM2831 |
| 143 | *ENSP00000255039* | *Hyaluronan and proteoglycan link protein 2 Precursor* | YM2843 |
| 144 | *ENSP00000380417* | *Interleukin-25 Precursor* | YM2854 |

In order to confirm the above results, the authors probed the focused protein microarray with a second serum sample set (*Test Set* as indicated in Table 1 *Material and Methods*) comprising other 15 sera of patients with AILD and 39 serum samples from healthy subjects. Following the same normalization and using criteria described above (see *Materials and Methods),* the authors confirmed that the 25 autoantigens were differentially recognized by patients compared to healthy donors with statistical significance. Interestingly, in unsupervised hierarchical clustering analysis these autoantigens allowed for good discrimination of the two populations of sera in both sample sets, as shown in Figure 4a.

Having identified 25 autoantigens highly recognized by AILD patients, the authors asked whether non-autoimmune liver disease patients had an overlapped recognition pattern. They therefore tested the same microarray with sera from 110 patients with chronic HCV infection (Table 1). Figure 4b shows the MFIs of the 25 autoantigens in each group of individuals (AILD, HD and HCV respectively), and highlights that, although some reactivity is observed in patients affected from HCV, 17 auto-antigens react preferentially and more strongly with sera from AILD patients, with statistical significance (p val <0.01).

### Example 4- Validation of selected autoantigens with an independent sample set confirms that 6 proteins are new potential AILD biomarkers.

After having identified a total of 17 auto-antigens specific for autoimmune patients by protein microarray, the authors validated the results obtained by using the Dissociation-Enhanced Lanthanide Fluorescence ImmunoAssay method (DELFIA^{®}) assay method (*Materials and Methods*)*.*

By this assay the authors screened an independent sample set (Validation set as indicated in Table 1, *Materials and Methods)* comprising 100 AILD patients (50 AIH and 50 PBC), 50 healthy donors and 74 patients with chronic viral hepatitis (50 HCV and 24 HBV) measured by time-resolved fluorescence. All sera were tested at a dilution of 1:300 as described in *Materials and Methods,* and the antigen-specific IgG responses to each of the 17 selected autoantigens was measured by time-resolved fluorescence. Reproducibility of the results was confirmed using duplicate sample of selected sera.

All 17 antigens displayed higher mean fluorescence intensity compared to HD and chronic viral hepatitis (Figure 5). Fluorescence intensity values higher than the mean plus three standard deviations of buffer signals among healthy donors were considered positive. As shown in Table 5, 6 of the 17 antigens displayed significantly higher recognition frequency by AILD patients compared to healthy donors, and were also significant when compared to viral hepatitis patients (Figure 6), thus being considered as top candidates.

**Table 5: Recognition frequencies of individual antigens identified as candidate AILD biomarkers in the validation sample set.**

| **Description** | **Prot. Id** | **Combo^{A}** | **Positive sera %** | | |
|---|---|---|---|---|---|
| | | | **HD (n=50)** | **AILD (n=100)** | **VH (n=74)** |
| *Asialoglycoprotein receptor* | AGPR | | 50 | 70 | 53 |
| *Cytochrome P4502D6* | Cyp450 | | 56 | 72 | 55 |
| *Pyruvate dehydrogenase* | PDH | | 0 | 55 | 4 |
| *Interleukin-4 receptor domain* | YM0078 | xo● | 2 | 74 | 14 |
| *Putative uncharacterized protein* | YM1503 | x | 6 | 48 | 14 |
| *Putative uncharacterized protein* | YM1602 | x | 8 | 44 | 14 |
| *Uncharacterized protein C17orf99* | YM1652 | xo | 4 | 47 | 15 |
| *Meteorin-like protein Precursor* | YM1672 | xo● | 0 | 48 | 22 |
| *Protein APC001-like Precursor* | YM2046 | xo● | 2 | 63 | 28 |

| | | | | | |
|---|---|---|---|---|---|
| HD: Healthy donors; AILD: autoimmune liver disease patients, VH: Viral hepatitis patients. ^{A}Antigens used for combination assays comprising 6 (x), 4 (o) and 3 (●) markers respectively. SE % = percentage of positive AIH sera; SP % = percentage of negative HD or VH sera. | | | | | |

These top 6 antigens, showed high sensitivity (from 44 to 74% of positive AILD patients) and specificity (from 92 to 100% of negative HD). Interestingly, individual sensitivity was comparable to that obtained in our hands by 3 known AILD markers, CYP2D6 (Cytochrome Peroxidase 2D6), AGPR-1 (Asialo-Glycoprotein Receptor 1) and PDH (Pyruvate DeHydrogenase), (Jensen, DM, 1978, The New England journal of medicine ; Van de Water J. et al., 1993, The Journal of clinical investigation), while individual specificity was far better for our candidates compared to the benchmarks.

The authors next assessed the discrimination power of combinations of the six autoantigens. We therefore tested combinations of three (YM0078, YM1672, YM2046), four (YM0078, YM1652, YM1672, YM2046) or six proteins (YM1672, YM0078, YM2046, YM1652, YM1503, YM1602). Figure 7a shows that combinations allow clear discrimination between patients with AILD and healthy donors, with sensitivities of 77%±4 (86, 81 and 79%, for 3, 4 and 6 combos) and specificities of 91%±8 (96, 92 and 82% respectively). Moreover, Figure 7b shows the ROC curves of logistic regression models obtained with the same combinations of new antigens compared to the combination of the three known control proteins, and illustrates that any of the new antigens combo is superior to the combo of the three known control proteins.

### REFERENCES

1. Czaja, A.J., and Manns, M.P. 2010. Gastroenterology 139:58-72 e54.
2. Makol, A., Watt, K.D., and Chowdhary, V.R. 2011. 2011:390916.
3. Bogdanos DP, Mieli-Vergani G, Vergani D. 2009. 29(3):241-53.
4. Selmi, C., Mackay, I.R., and Gershwin, M.E. 2011. 89:70-80.
5. Muratori L, Muratori P, Granito A, Pappas G, Cassani F, Lenzi M. 2010. Dig Liver Dis 42(11):757-64.
6. Muratori, L., Granito, A., Muratori, P., Pappas, G., and Bianchi, F.B. 2008. Clinics in liver disease 12:261-276; vii.
7. Bogdanos, D.P., Invernizzi, P., Mackay, I.R., and Vergani, D. 2008. World journal of gastroenterology : WJG 14:3374-3387.
8. Vergani, D., Alvarez, F., Bianchi, F.B., Cancado, E.L., Mackay, I.R., Manns, M.P., Nishioka, M., and Penner, E. 2004. Journal of hepatology 41:677-683.
9. Zachou, K., Rigopoulou, E., and Dalekos, G.N. 2004. Journal of autoimmune diseases 1:2.
10. Song, Q., Liu, G., Hu, S., Zhang, Y., Tao, Y., Han, Y., Zeng, H., Huang, W., Li, F., Chen, P., et al. 2010. 9:30-39.
11. Grifantini, R., Pagani, M., Pierleoni, A., Grandi, A., Parri, M., Campagnoli, S., Pileri, P., Cattaneo, D., Canidio, E., Pontillo, A., et al. 2011. Journal of proteomics 75:532-547.
12. Bombaci, M., Grifantini, R., Mora, M., Reguzzi, V., Petracca, R., Meoni, E., Balloni, S., Zingaretti, C., Falugi, F., Manetti, A.G., et al. 2009. PloS one 4:e6332.
13. Harris, J. W. and Stocker, H. 1998. Handbook of Mathematics and Computational Science. New York: Springer-Verlag.
14. Saeed, A.I., Bhagabati, N.K., Braisted, J.C., Liang, W., Sharov, V., Howe, E.A., Li, J., Thiagarajan, M., White, J.A., and Quackenbush, J. 2006. Methods in enzymology 411:134-193.
15. Frulloni, L., Lunardi, C., Simone, R., Dolcino, M., Scattolini, C., Falconi, M., Benini, L., Vantini, I., Corrocher, R., and Puccetti, A. 2009. The New England journal of medicine 361:2135-2142.
16. Sing, T., Sander, O., Beerenwinkel, N., and Lengauer, T. 2005. ROCR: visualizing classifier performance in R. Bioinformatics 21:3940-3941.
17. Jensen, D.M., McFarlane, I.G., Portmann, B.S., Eddleston, A.L., and Williams, R. 1978. The New England journal of medicine 299:1-7.
18. Van de Water, J., Turchany, J., Leung, P.S., Lake, J., Munoz, S., Surh, C.D., Coppel, R., Ansari, A., Nakanuma, Y., and Gershwin, M.E. 1993. The Journal of clinical investigation 91:2653-2664.

## Claims

1. An *in vitro* method of diagnosis or prognosis or evaluation of risk to develop a liver autoimmune disorder belonging to the group of autoimmune hepatitis (AIH) and primary biliary cirrhosis (PBC) in a subject, comprising the steps of:
a) contacting a biological sample from the subject with a protein having the amino acid sequence SEQ ID No. 1, under conditions appropriate for binding of autoantibodies, if present in the biological sample, to said protein, and
b) detecting the presence of bound autoantibodies.

2. The method according to claim 1 wherein step a) is performed by contacting said biological sample with the protein as in claim 1 and at least one further protein selected from the group of 16 proteins having the amino acid sequences SEQ ID No. 2, 3, 5, 6, 7, 8, 9, 10, 11, 12, 14, 15, 16, 17, 22, 25.

3. The method according to claim 2 wherein step a) is performed by contacting said biological sample with three proteins having the amino acid sequences SEQ ID No. 1, 11, 17.

4. The method according to claim 3 wherein step a) is performed by contacting said biological sample with four proteins having the amino acid sequences SEQ ID No. 1, 10, 11, 17.

5. The method according to claim 4 wherein step a) is performed by contacting said biological sample with six proteins having the amino acid sequences SEQ ID No. 1, 6, 8, 10,11,17.

6. The method according to any of previous claims wherein the biological sample is comprised in the group of blood, serum, plasma, urine, saliva, mucus, or fractions thereof.

7. The method of any one of previous claims wherein the biological sample is from an adult or from an adolescent.

8. The method according to any of previous claims wherein the detection of said bound autoantibodies is performed by means of binding to specific ligands.

9. The method according to claim 8 wherein the ligands are conjugated with detecting means.

10. Use of a protein microarray comprising at least the proteins as defined in any of claims 1 to 5 for performing the method according to any of previous claims.

11. Use of a solid support for an immunodiagnostic assay comprising at least the proteins as defined in any of claims 1 to 5 for performing the method according to any of previous claims 1 to 9.

## Patentansprüche

1. In-vitro-Verfahren der Diagnose oder Prognose oder Evaluierung eines Risikos zur Entwicklung einer Autoimmunstörung der Leber, die zu der Gruppe Autoimmunhepatitis (AIH) und primäre biliäre Zirrhose (PBC) zählt, in einem Lebewesen, das folgende Schritte aufweist:
a) In-Berührung-bringen einer biologischen Probe aus dem Lebewesen mit einem Protein, das die Aminosäuresequenz SEQ ID NR. 1 aufweist, unter Bedingungen, die für eine Bindung von Autoantikörpern, sofern sie in der biologischen Probe vorhanden sind, an das Protein geeignet sind, und
b) Detektieren des Vorhandenseins gebundener Autoantikörper.

2. Verfahren nach Anspruch 1, wobei Schritt a) durchgeführt wird, indem die biologische Probe mit dem Protein nach Anspruch 1 und mindestens einem weiteren Protein, das aus der Gruppe von 16 Proteinen mit den Aminosäuresequenzen SEQ ID NR. 2, 3, 5, 6, 7, 8, 9, 10, 11, 12, 14, 15, 16, 17, 22, 25 ausgewählt ist, in Berührung gebracht wird.

3. Verfahren nach Anspruch 2, wobei Schritt a) durchgeführt wird, indem die biologische Probe mit drei Proteinen mit den Aminosäuresequenzen SEQ ID Nr. 1, 11, 17 in Berührung gebracht wird.

4. Verfahren nach Anspruch 3, wobei Schritt a) durchgeführt wird, indem die biologische Probe mit vier Proteinen mit den Aminosäuresequenzen SEQ ID Nr. 1, 10, 11, 17 in Berührung gebracht wird.

5. Verfahren nach Anspruch 4, wobei Schritt a) durchgeführt wird, indem die biologische Probe mit sechs Proteinen mit den Aminosäuresequenzen SEQ ID Nr. 1, 6, 8, 10, 11, 17 in Berührung gebracht wird.

6. Verfahren nach einem der vorhergehenden Ansprüche, wobei die biologische Probe in der Gruppe aus Blut, Serum, Plasma, Urin, Speichel, Mukus oder Fraktionen davon enthalten ist.

7. Verfahren nach einem der vorhergehenden Ansprüche, wobei die biologische Probe von einem Erwachsenen oder von einem Jugendlichen stammt.

8. Verfahren nach einem der vorhergehenden Ansprüche, wobei die Detektion der gebundenen Autoantikörper mittels Bindung an spezifische Liganden durchgeführt wird.

9. Verfahren nach Anspruch 8, wobei die Liganden mit detektierenden Mitteln konjugiert sind.

10. Verwendung eines Protein-Mikroarrays, das mindestens die Proteine nach der Definition in einem der Ansprüche 1 bis 5 aufweist, zum Durchführen des Verfahrens nach einem der vorhergehenden Ansprüche.

11. Verwendung eines festen Trägers für einen immundiagnostischen Assay, der mindestens die Proteine nach der Definition in einem der Ansprüche 1 bis 5 aufweist, zum Durchführen des Verfahrens nach einem der vorhergehenden Ansprüche 1 bis 9.

## Revendications

1. Procédé *in vitro* de diagnostic ou de pronostic ou d'évaluation du risque de développer un trouble hépatique auto-immun appartenant au groupe de l'hépatite auto-immune (HAI) et de la cirrhose biliaire primaire (CBP) chez un sujet, comprenant les étapes de :
a) mise en contact d'un échantillon biologique prélevé sur le sujet avec une protéine ayant la séquence d'acides aminés SEQ ID NO : 1, dans des conditions adaptées à la liaison d'auto-anticorps, s'ils sont présents dans l'échantillon biologique, à ladite protéine, et
b) détection de la présence d'auto-anticorps liés.

2. Procédé selon la revendication 1, dans lequel l'étape a) est réalisée par mise en contact dudit échantillon biologique avec la protéine selon la revendication 1 et au moins une autre protéine sélectionnée dans le groupe constitué de 16 protéines ayant les séquences d'acides aminés SEQ ID NO : 2, 3, 5, 6, 7, 8, 9, 10, 11, 12, 14, 15, 16, 17, 22, 25.

3. Procédé selon la revendication 2, dans lequel l'étape a) est réalisée par mise en contact dudit échantillon biologique avec trois protéines ayant les séquences d'acides aminés SEQ ID NO : 1, 11, 17.

4. Procédé selon la revendication 3, dans lequel l'étape a) est réalisée par mise en contact dudit échantillon biologique avec quatre protéines ayant les séquences d'acides aminés SEQ ID NO : 1, 10, 11, 17.

5. Procédé selon la revendication 4, dans lequel l'étape a) est réalisée par mise en contact dudit échantillon biologique avec six protéines ayant les séquences d'acides aminés SEQ ID NO : 1, 6, 8, 10, 11, 17.

6. Procédé selon l'une quelconque des revendications précédentes, dans lequel l'échantillon biologique est compris dans le groupe du sang, du sérum, du plasma, de l'urine, de la salive, du mucus, ou de fractions de ceux-ci.

7. Procédé selon l'une quelconque des revendications précédentes, dans lequel l'échantillon biologique est prélevé sur un adulte ou sur un adolescent.

8. Procédé selon l'une quelconque des revendications précédentes, dans lequel la détection desdits auto-anticorps liés est réalisée au moyen de la liaison à des ligands spécifiques.

9. Procédé selon la revendication 8, dans lequel les ligands sont conjugués à un moyen de détection.

10. Utilisation d'une micropuce à protéines comprenant au moins les protéines selon l'une quelconque des revendications 1 à 5, pour réaliser le procédé selon l'une quelconque des revendications précédentes.

11. Utilisation d'un support solide pour un test immunodiagnostique comprenant au moins les protéines selon les revendications 1 à 5 pour la réalisation du procédé selon l'une quelconque des revendications 1 à 9 précédentes.
